(19) Europäisches Patentamt
European Patent Office
Office européen des brevets

(11) EP 0 743 312 B1

(12) **FASCICULE DE BREVET EUROPEEN**

(45) Date de publication et mention de la délivrance du brevet:
**17.11.1999 Bulletin 1999/46**

(51) Int Cl.[6]: **C07D 498/10**, A61K 31/435, A61K 31/47
// (C07D498/10, 263:00, 221:00)

(21) Numéro de dépôt: **96401029.2**

(22) Date de dépôt: **13.05.1996**

(54) **Nouveaux composés spiro hétérocycliques, leur procédé de préparation et les compositions pharmaceutiques les contenant**

Spiroheterozyklische Verbindungen, Verfahren zu ihrer Herstellung und diese enthaltende pharmazeutische Zusammensetzungen

Spiroheterocyclic compounds, process for their preparation and pharmaceutical compositions containing them

(84) Etats contractants désignés:
**AT BE CH DE DK ES FI FR GB GR IE IT LI LU NL PT SE**

(30) Priorité: **17.05.1995 FR 9505833**

(43) Date de publication de la demande:
**20.11.1996 Bulletin 1996/47**

(73) Titulaire: **ADIR ET COMPAGNIE**
**92415 Courbevoie Cédex (FR)**

(72) Inventeurs:
- **Guillonneau, Claude**
  **92140 Clamart (FR)**
- **Charton, Yves**
  **92330 Sceaux (FR)**
- **Regnier, Gilbert**
  **92290 Chatenay Malabry (FR)**
- **Canet, Emmanuel**
  **75014 Paris (FR)**
- **Lonchampt, Michel**
  **94150 Chevilly Larue (FR)**

(74) Mandataire: **Reverbori, Marcelle**
**ADIR**
**1, rue Carle Hébert**
**92415 Courbevoie Cédex (FR)**

(56) Documents cités:
**EP-A- 0 479 631** **US-A- 3 399 192**

- **CHEMICAL AND PHARMACEUTICAL BULLETIN., vol. 33, no. 3, 1985, TOKYO JP, pages 1129-1139, XP002010391 H. TAKAI ET AL: "Spiropiperidines. I. Synthesis of 1'-substituted spiro(4H-3,1-benzoxazine-4,4'-piperidin)-2 (1H)-one derivatives and evaluation of their antihypertensive activity"**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

EP 0 743 312 B1

Printed by Jouve, 75001 PARIS (FR)

## Description

La présente invention a pour objet de nouveaux composés spiro hétérocycliques, leur procédé de préparation et les compositions pharmaceutiques les contenant.
Elle concerne particulièrement les composés spiro hétérocycliques de formule I :

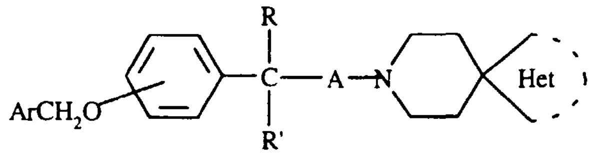

(I)

dans laquelle :

- Ar représente :

  a) un radical hydrocarboné aromatique mono- ou bi-cyclique, éventuellement substitué par un ou plusieurs substituants choisis parmi les atomes d'halogène et les radicaux $(C_1-C_5)$alkyle, $(C_1-C_5)$alkoxy, trifluorométhyle et phényle, ou
  b) un radical hétérocyclique mono- ou bi-cyclique renfermant de 1 à 3 hétéroatomes choisis parmi les atomes d'oxygène, azote et soufre et éventuellement substitué par un ou plusieurs substituants choisis parmi les atomes d'halogène et les radicaux $(C_1-C_5)$alkyle, $(C_1-C_5)$alkoxy, trifluorométhyle et phényle ;

- R représente un atome d'hydrogène ou un radical hydroxy ;
- R' représente un atome d'hydrogène ou un radical choisi parmi les radicaux $(C_1-C_5)$alkyle en chaîne droite ou ramifiée, phényle, phényl-$(C_1-C_5)$alkyle, $(C_3-C_8)$cycloalkyle et $(C_3-C_8)$cycloalkyl-$(C_1-C_5)$alkyle, chacun de ces radicaux étant soit non substitué, soit substitué par un ou plusieurs substituants choisis parmi les atomes d'halogène et les radicaux $(C_1-C_5)$alkyle, $(C_1-C_5)$alkoxy et trifluorométhyle ; ou
- R et R' représentent ensemble avec l'atome de carbone auquel ils sont liés un groupe carbonyle ;
- A représente :

  . une liaison simple,
  . un groupe carbonyle, ou
  . une chaîne hydrocarbonée droite de 1 à 5 atomes de carbone, qui peut contenir éventuellement un atome d'oxygène, et/ou être éventuellement substituée par un ou deux substituants, choisis parmi les atomes d'halogène, et les radicaux $(C_1-C_5)$alkyle, $(C_1-C_5)$alkoxy et oxo ; et

-

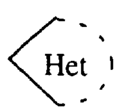

représente un hétérocycle pentagonal contenant de 1 à 4 hétéroatomes choisis parmi les atomes d'oxygène, azote et soufre, lequel hétérocycle est soit non substitué, soit substitué par un ou deux substituants choisis parmi les radicaux $(C_1-C_5)$alkyle, $(C_1-C_5)$alkoxy, hydroxy, oxo, thio, amino, thioxo, amino-$(C_1-C_5)$alkyle, $(C_1-C_5)$alkylamino-$(C_1-C_5)$alkyle et di$(C_1-C_5)$alkylamino$(C_1-C_5)$alkyle.

Certains composés de formule I contiennent un ou plusieurs atomes asymétriques et peuvent ainsi exister sous forme d'énantiomères ou de diastéréoisomères qui font également partie de la présente invention.
De même, les composés de formule I contenant une ou plusieurs fonctions amine, peuvent être transformés en sels d'addition avec des acides pharmaceutiquement acceptables, comme par exemple les acides chlorhydrique et fumarique. Ces sels sont, à ce titre, inclus dans la présente invention.
L'état antérieur de la technique le plus proche de la présente invention est illustré par le brevet US 3 399 192 qui concerne les composés du spiro(4,5)décane de formule :

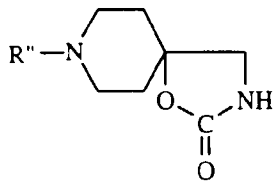

dans laquelle R" représente, entre autres, un radical phénylalkyle dont :

- la partie phényle peut être substituée mais jamais par le radical $ArCH_2O$- présent dans la formule I, et
- la partie alkyle peut éventuellement renfermer un atome d'oxygène ou un radical hydroxy.

Ces dits composés du spiro(4,5)décane sont des agents antagonistes de certains médiateurs chimiques comme la sérotonine, l'histamine et la bradykinine.

Les composés de la présente invention diffèrent des composés du spiro(4,5)décane ci-dessus définis, à la fois par leur structure chimique et par leur activité pharmacologique et thérapeutique, laquelle découle de leur effet inhibiteur de l'enzyme 5-lipoxygénase.

La présente invention a également pour objet le procédé de préparation des composés de formule I caractérisé en ce que l'on fait réagir :

A) un composé de formule II :

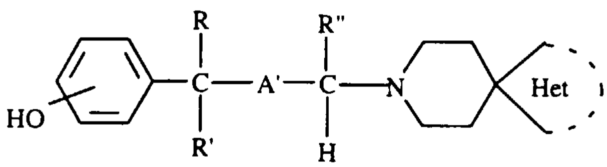

(II)

dans laquelle :

- R, R' et

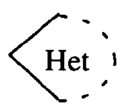

ont les significations précédemment définies,
- A' représente :

  . une liaison simple, ou
  . une chaîne hydrocarbonée droite de 1 à 4 atomes de carbone qui peut contenir éventuellement un atome d'oxygène et/ou être éventuellement substituée par un ou plusieurs substituants choisis parmi les atomes d'halogène et les radicaux $(C_1\text{-}C_5)$alkyle, $(C_1\text{-}C_5)$alkoxy, hydroxy et oxo,

- R" représente un atome d'hydrogène ou un radical $(C_1\text{-}C_4)$alkyle linéaire ou ramifié ;
avec un composé de formule III:

$$Ar\text{-}CH_2\text{-}X \qquad (III)$$

dans laquelle :
- Ar a la signification précédemment définie, et
- X représente un atome d'halogène, tel qu'un atome de chlore ou de brome ;
pour obtenir un composé de formule Ia :

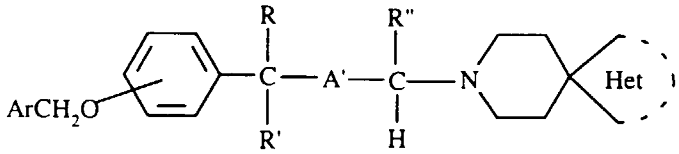
(Ia)

dans laquelle Ar, R, R', A', R" et

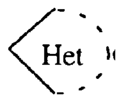

ont les significations précédemment définies ; ou

B) un composé de formule IV :

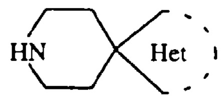
(IV)

dans laquelle

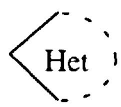

a la signification précédemment définie ;

a) soit avec un composé de formule V :

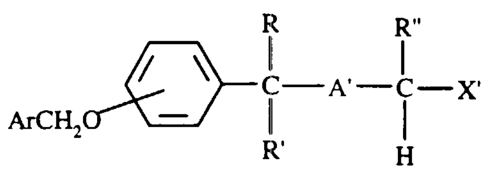
(V)

dans laquelle :

- Ar, R, R', A' et R" ont les significations précédemment définies, et
- X' représente un atome d'halogène, tel que par exemple un atome de brome ou de chlore, ou un groupe tosyloxy ou mésyloxy,

pour obtenir un composé de formule Ia précédemment définie ;
b) soit avec un composé de formule VI :

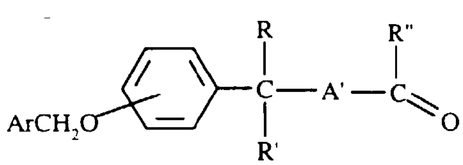
(VI)

dans laquelle Ar, R, R', A' et R" ont les significations précédemment définies,
en présence d'un agent réducteur tel que par exemple un hydrure de bore,

pour obtenir un composé de formule Ia précédemment définie,
c) soit avec un composé de formule VII :

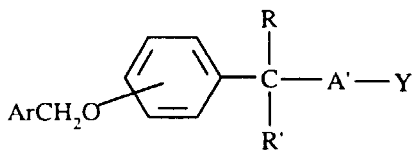
(VII)

dans laquelle :

- Ar, R, R' et A' ont les significations précédemment définies, et
- Y représente -COOH ou COCl,

pour obtenir un composé de formule Ib :

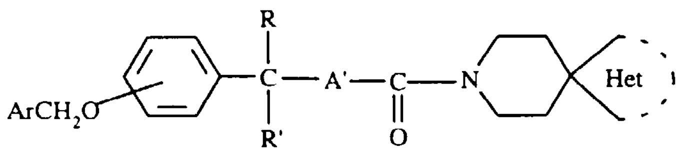
(Ib)

dans laquelle Ar, R, R', A' et

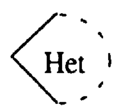

ont les significations précédemment définies ;
d) soit avec un composé de formule VIII:

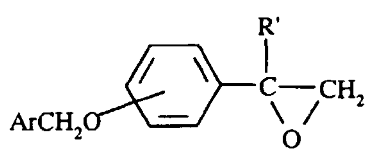
(VIII)

dans laquelle Ar et R' ont les significations précédemment définies,
pour obtenir un composé de formule Ic :

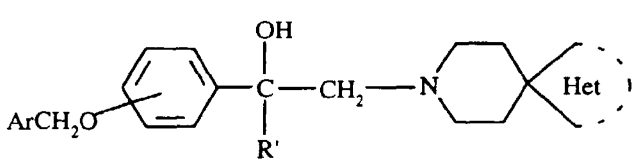
(Ic)

dans laquelle Ar, R' et

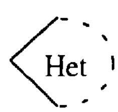

ont les significations précédemment définies ;

L'ensemble des composés de formule Ia, Ib et Ic forme l'ensemble des composés de formule I.

Il est particulièrement avantageux de faire réagir les composés de formule II et III dans un solvant comme par exemple la méthyléthylcétone, le diméthylformamide ou l'acétonitrile à une température comprise entre 50 et 100 °C, en présence d'un accepteur de l'hydracide formé au cours de la réaction.

Comme accepteur d'hydracide, on peut utiliser par exemple un carbonate alcalin tel que le carbonate de potassium en présence d'un iodure alcalin ou la triéthylamine.

La réaction des composés IV et V s'effectue avantageusement dans un solvant comme, par exemple, l'acétone, l'acétonitrile ou le diméthylformamide, à une température comprise entre 50 et 120 °C, en présence d'un accepteur de l'hydracide formé au cours de la réaction. Comme accepteur, on peut utiliser par exemple un excès du composé de formule IV, la diméthylaminopyridine ou la triéthylamine.

La réaction des composés de formule IV et VI peut s'effectuer dans un solvant aprotique tel que le dichlorométhane ou le tétrahydrofurane en présence d'un réducteur tel que le triacétoxyborohydrure de sodium, à une température comprise entre 15 et 45 °C.

Il est également possible de condenser les composés de formule IV et VI dans un solvant aromatique tel que le toluène en présence d'un catalyseur acide tel que l'acide 4-méthylphénylsulfonique. L'énamine intermédiaire peut être réduite soit par un réducteur chimique tel que le cyanoborohydrure de sodium, soit par hydrogénation dans un alcool à bas point d'ébullition, tel que l'éthanol, en présence d'un catalyseur comme le palladium sur charbon ou le nickel de Raney.

La réaction des composés de formule IV et VII s'effectue :

- soit, lorsque Y représente COOH, en présence d'un agent de couplage tel que la dicyclohexylcarbodiimide dans le diméthylformamide, ou l'anhydride cyclique de l'acide 1-propylphosphonique selon la technique de H. Wissmann et H.J. Kleiner, Angew. Chem. Int. Ed. Engl. 19 (1980) n° 2 p 133 ;
- soit lorsque Y représente COCl, en présence d'un accepteur d'hydracide tel que la triéthylamine ou la diméthylaminopyridine, dans un solvant aprotique tel que l'éther, le tétrahydrofurane, le dichlorométhane, ou le diméthylformamide ou dans un solvant basique tel que la pyridine.

La réaction des composés de formule IV et VIII s'effectue avantageusement dans un alcool à bas point d'ébullition tel que le méthanol ou l'éthanol, à une température comprise entre 40 et 100 °C, éventuellement en présence d'un acide de Lewis tel que le trifluorure de bore.

Les matières premières utilisées sont soit des produits connus soit des produits préparés par des méthodes classiques à partir de composés connus.

Ainsi, les composés de formule II sont obtenus :

- soit en faisant réagir un composé de formule IX :

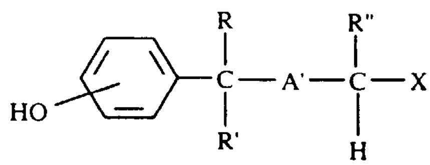

(IX)

dans laquelle R, R', R" et X ont les significations précédemment définies avec un composé de formule IV précédemment définie, dans un solvant aprotique polaire, tel que par exemple l'acétonitrile ou la méthyléthylcétone en présence d'un accepteur d'hydracide qui peut être un excès du composé de formule IV ou un carbonate alcalin ;
- soit en déprotégeant un composé de formule X :

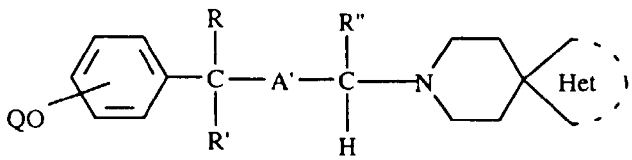

(X)

dans laquelle :

- R,R',R",A' et

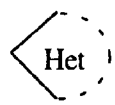

ont les significations précédemment définies et

- Q est un groupe protecteur labile comme par exemple un radical méthyle ou benzyle.

La déprotection peut s'effectuer en présence d'un acide de Lewis, tel que le tribromure de bore, dans un solvant aprotique, comme le dichlorométhane.

Lorsque Q est un radical benzyle, la déprotection peut également être effectuée par hydrogénation en présence d'un catalyseur d'hydrogénation tel que le palladium sur charbon ou l'hydroxyde de palladium sur charbon dans un alcool à bas point d'ébullition, comme par exemple l'éthanol.

Les composés de formule III sont des produits connus décrits dans la littérature et généralement commercialisés.

Les composés de formule IV utilisés dans la synthèse des composés exemplifiés ont été préparés selon des méthodes décrites dans la littérature et repertoriées dans le tableau ci-après :

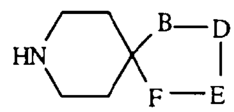

| *B* | *D* | *E* | *F* | *Caractéristiques physiques* | *Méthode de préparation* |
|---|---|---|---|---|---|
| $CH_2$ | N-H | C=O | O | $PF_{(K)}$ : 202 °C | G. REGNIER et coll. Chimie Thérapeutique (1969) (3), 185-194 |
| 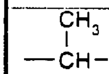 | N-H | C=O | O | $PF_{(cap)}$ : : 245-246 °C | J. MAILLARD et coll. Chimie Thérapeutique (1973)(4), 393,397 |
| O | $CH_2$ | $CH_2$ | O | | Débenzylation du dérivé 8-benzylé correspondant |
| $CH_2$ | O | C=O | NH | Chlorhydrate $PF_{(cap)}$ : 217-218 °C | Débenzylation du dérivé 8-benzylé correspondant. $PF_{(cap)}$ : 168-170 °C |
| $CH_2$ | 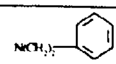 | C=O | O | Chlorhydrate $PF_{(cap)}$ : 246-247°C | Débenzylation du dérivé 8-benzylé correspondant. $PF_{(K)}$ : 153 °C |
| $CH_2$ | $N-CH_3$ | C=O | O | Chlorhydrate $PF_{(cap)}$ : 260 °C | Débenzylation du dérivé 8-benzylé correspondant. $PF_{(cap)}$ : 130 °C |
| $CH_2$ | $N-C_2H_5$ | C=O | O | Chlorhydrate $PF_{(cap)}$ : 230 °C | Débenzylation du dérivé 8-benzylé correspondant. $PF_{(K)}$ : 95 °C |
| $CH_2$ | $N-(CH_2)_2-N(CH_3)_2$ | C=O | O | Dichlorhydrate $PF_{(cap)}$ : 250 °C (déc) | Débenzylation du dérivé 8-benzylé correspondant. $PF_{(cap)}$ : 101 °C |

| *B* | *D* | *E* | *F* | *Caractéristiques physiques* | *Méthode de préparation* |
|---|---|---|---|---|---|
| $CH_2$ | C=O | NH | C=O | $PF_{(K)}$ : 245 °C | Débenzylation du dérivé 8-benzylé correspondant. $PF_{(K)}$ : 187 °C |
| $CH_2$ | C=O | NH | $CH_2$ | Chlorhydrate $PF_{(cap)}$ : 250 °C (déc) | Débenzylation du dérivé 8-benzylé correspondant. $PF_{(cap)}$ : 152 °C G. CIGNARELLA S. VILLA J. Heterocyclic Chemistry (1993), vol. 30, N° 5, 1357. |
| $CH_2$ | NH | C=S | O | $PF_{(K)}$ : 241 °C | Détritylation du dérivé 8-tritylé correspondant. $PF_{(cap)}$ : 248-250 °C |

Les composés de formule V ont été obtenus en faisant réagir un composé de formule XI :

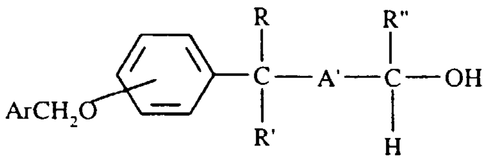

(XI)

dans laquelle Ar, R, R', A' et R" ont les significations précédemment définies, avec un réactif d'halogénation comme le chlorure de thionyle, ou le pentachlorure de phosphore ou avec la triphénylphosphine, en présence de $CCl_4$, ou de brome dans l'acétonitrile selon la méthode de J. Hooz et coll, Can. J. Chem. 46, 86-87 (1968) ou de Schaeffer et coll, Org. Synth. coll. vol V, 249, ou avec un halogénure d'acide sulfonique comme le chlorure de tosyle dans un solvant basique tel que la pyridine, à une température comprise entre 5 et 25 °C.

Les composés de formule XI sont eux-mêmes obtenus en faisant réagir un composé de formule XII:

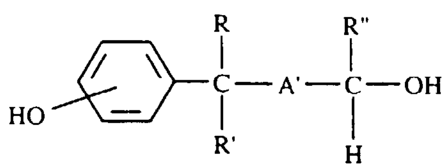

(XII)

dans laquelle R, R', A' et R" ont les significations précédemment définies, avec un composé de formule III précédemment définie.

Une telle réaction peut s'effectuer :

- soit dans des amides ou des cétones à bas poids moléculaires, comme par exemple le diméthylformamide ou la méthyléthylcétone, en présence d'un carbonate alcalin, tel que le carbonate de potassium, ou d'une base organique, telle que la triéthylamine, à une température comprise entre 10 et 70 °C,
- soit dans le dichlorométhane, en présence d'eau, d'un carbonate alcalin, tel que le carbonate de potassium, et d'un halogénure d'ammonium quaternaire, tel que l'Adogène® à une température comprise entre 10 et 40 °C.

En ce qui concerne les composés de formule VI utilisables pour synthétiser les composés ci-après exemplifiés :
les composés de formule VIa:

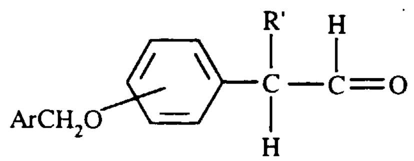

(VIa)

dans laquelle Ar et R' ont les significations précédemment définies,
ont été obtenus par isomérisation en présence d'un acide de Lewis, d'un composé de formule VIII précédemment définie, selon la technique décrite par E.J. Corey et M. Chaykovsky, J. Am. Chem. Soc. 87, 6, (1965).

Les composés de formule VIII sont eux-mêmes obtenus en faisant réagir un composé de formule XIII :

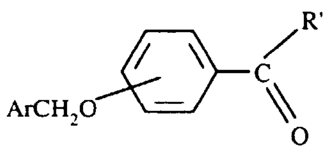

(XIII)

dans laquelle Ar et R' ont les significations précédemment définies,
avec l'iodure de triméthylsulfonium ou l'iodure de triméthylsulfoxonium dans le diméthylsulfoxyde, en présence d'hydrure de sodium, selon la technique décrite par E.J. Corey et M. Chaykovsky, J Am. Chem. Soc. 87, 6, (1965).

Les composés de formule XIII sont eux obtenus en faisant réagir un composé de formule XIV :

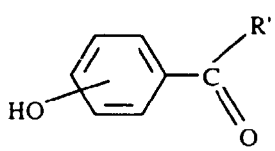

(XIV)

dans laquelle R' a la signification précédemment définie (lequel est, selon la valeur de R', soit commercial soit décrit dans la littérature) avec un composé de formule III précédemment définie,
dans un solvant aprotique polaire tel que le diméthylformamide, en présence d'un accepteur d'hydracide tel que le carbonate de potassium ;
les composés de formule VIb :

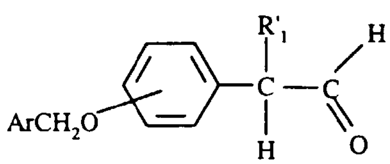

(VIb)

dans laquelle :

- Ar a la signification précédemment définie, et
- R'$_1$ représente un atome d'hydrogène ou un radical choisi parmi les radicaux phényl-($C_1$-$C_5$)alkyle, ($C_3$-$C_8$)cycloalkyle et ($C_3$-$C_8$)cycloalkyl-($C_1$-$C_5$) alkyle, chacun de ces radicaux étant soit non substitué, soit substitué par un ou plusieurs substituants choisis parmi les atomes d'halogène et les radicaux ($C_1$-$C_5$)alkyle, ($C_1$-$C_5$)alkoxy et trifluorométhyle, sont obtenus en faisant réagir un composé de formule XV :

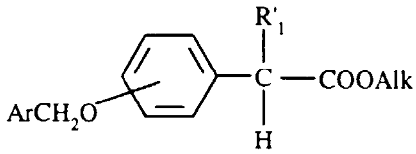

(XV)

dans laquelle :

- Ar et $R'_1$ ont les significations précédemment définies et
- Alk représente un radical ($C_1$ -$C_5$)alkyle,

avec l'hydrure de diisobutyl aluminium selon la technique de Muraki et Mukaiyama, Chem. Lett. (1975), 215.

Parmi les composés de formule XV, ceux pour lesquels $R'_1$ prend la valeur hydrogène, c'est à dire ceux répondant à la formule XVa :

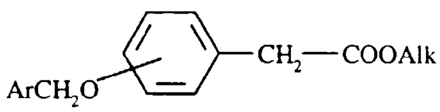

(XVa)

dans laquelle Ar et Alk ont les significations précédemment définies,
ont été obtenus en faisant réagir les composés de formule XVI :

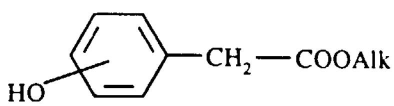

(XVI)

dans laquelle Alk a la signification précédemment définie,
(lesquels composés XVI sont des produits connus)
avec un composé de formule III précédemment définie, en opérant dans un solvant aprotique polaire tel que la diméthylformamide, en présence d'un accepteur d'hydracide comme le carbonate de potassium, les composés de formule XV, pour lesquels $R'_1$ ne prend pas la valeur hydrogène, c'est à dire ceux répondant à la formule XVb :

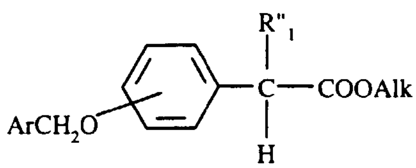

(XVb)

dans laquelle :

- Ar et alk ont les significations précédemment définies et
- $R''_1$ a la même signification que $R'_1$ à l'exception de la valeur hydrogène,

sont obtenus en faisant réagir un composé de formule XVa précédemment définie,
avec un halogénure de formule XVII :

$$R''_1\text{-X} \qquad \text{(XVII)}$$

dans laquelle $R''_1$ et X ont les significations précédemment définies en opérant en présence d'une base forte telle que par exemple l'amidure de sodium dans un solvant approprié comme par exemple le toluène.

Les composés de formule VII ont été synthétisés à partir des composés de formule XVIII :

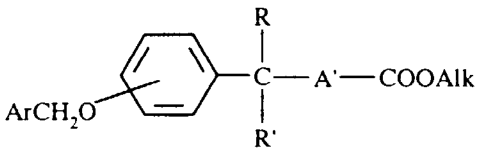 (XVIII)

dans laquelle Ar, R, R', A' et Alk ont les significations précédemment définies,
qui, saponifiés à l'aide d'un hydroxyde alcalin, comme par exemple la soude, donnent les composés de formule VIIa :

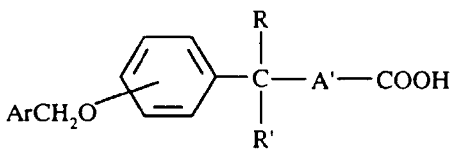 (VIIa)

dans laquelle Ar, R, R' et A' ont les significations précédemment définies,
lesquels, traités par un réactif classique de transformation d'un acide carboxylique en halogénure d'acide, comme par exemple le chlorure de thionyle ou le pentachlorure de phosphore, donnent les composés de formule VIIb :

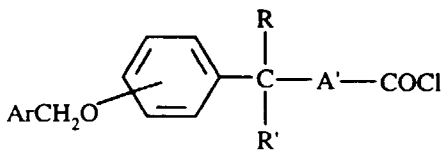 (VIIb)

dans laquelle Ar, R, R' et A' ont les significations précédemment définies.

L'ensemble des composés de formule VIIa et VIIb forme l'ensemble des composés de formule VII.

Les composés de formule XVIII ont eux-même été synthétisés en faisant réagir un composé de formule XIX :

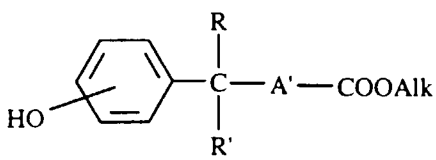 (XIX)

dans laquelle R, R', A' et Alk ont les significations précédemment définies, avec un composé de formule III précédemment définie dans un solvant aprotique polaire tel que le diméthylformamide en présence d'un accepteur d'hydracide tel que le carbonate de potassium.

Les composés de formule I ainsi obtenus peuvent être purifiés soit par chromatoflash sur silice (Amicon 35-70μ) en utilisant comme éluant, l'acétate d'éthyle ou un mélange de dichlorométhane et de méthanol, soit par formation de sels et cristallisation de ceux-ci.

Certains dérivés de formule I donnent des sels physiologiquement tolérables -sels qui sont, à ce titre, inclus dans la présente invention.

Les composés de la présente invention possèdent des propriétés pharmacologiques et thérapeutiques intéressantes.

En particulier, pour ces composés, a été mise en évidence in vitro et ex vivo une activité inhibitrice sur l'enzyme 5-Lipoxygénase.

La 5-Lipoxygénase est l'enzyme de la première étape du métabolisme de l'acide arachidonique conduisant à la synthèse des leucotriènes. Cette première étape aboutie à la synthèse du 5-Hydroperoxyeïcosatétraénoïque (5 (S)-HPETE) qui est converti en époxyde instable, le leucotriène A4 (LTA4). Le LTA4 est converti par hydratation enzymatique en leucotriène B4, ou par conjugaison au glutathion en leucotriène C4 qui par clivages protéolytiques successifs conduira à la formation de LTD4 et de LTE4 (cf. *Samuelsson B. and C.D. Funk. Enzymes Involved in the Biosynthesis of Leukotrienes B4. The Journal of Biochemistry. Vol. 264, N° 33, p. 19469-19472, 1989).* Les leucotriènes précités sont des substances lipidiques qui jouent un rôle important dans la physiopathologie de maladies diverses.

L'inhibition de la 5-Lipoxygénase, première étape conduisant la synthèse des leucotriènes, constitue ainsi une approche thérapeutique visant à limiter les effets de ces lipides. Des composés ayant des activités inhibitrices 5-Lipoxygénase sont donc utilisables dans le traitement des maladies humaines dans lesquelles le rôle des leucotriènes a été mentionné, comme notamment dans les pathologies dermatologiques telles que eczéma et psoriasis (cf. *Lewis R.A., Austen K.F. and R.J. Sobennan. Leukotrienes and Other Products of the 5-Lipoxygenase Pathway. The New England Journal of Medecine. Vol. 323, N° 10, p 645-655, 1990.)*

La présente invention a également pour objet les compositions pharmaceutiques contenant comme principe actif un composé de formule I, ou un de ses sels physiologiquement tolérable, mélangé ou associé à un ou plusieurs excipients pharmaceutiques appropriés comme par exemple le glucose, le lactose, l'amidon, l'éthylcellulose, le stéarate de magnésium ou le beurre de cacao.

Ces compositions pharmaceutiques se présentent généralement sous forme dosée. Elle peuvent être administrées à des doses thérapeutiques s'échelonnant de 0,001 mg à 25 mg de principe actif par kg de poids corporel par voie intraveineuse et de 0,01 mg à 100 mg de principe actif par kg de poids corporel par voie orale.

Les exemples suivants illustrent la présente invention, les points de fusion étant déterminés à la platine chauffante de Kofler (K) ou au tube capillaire (cap).

**Exemple 1**

8-{2-[3-(quinol-2-yl méthoxy)phényl]éthyl}-3,8-diaza-1-oxa-2-oxo spiro[4,5]décane :

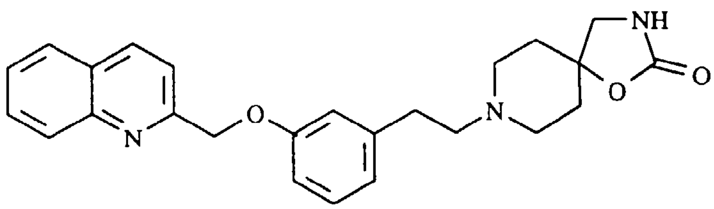

Dans un ballon on introduit 12,5 g (0,058 mole) de chlorhydrate de 2-chlorométhyl quinoléine, 9,8 g de 3-hydroxyphényl éthanol, 8 g de carbonate de potassium, 8 g de soude, 150 ml de dichlorométhane et 5 g d'Adogène 624. On agite 16 heures à température ambiante. On lave à l'eau, sèche sur sulfate de sodium et concentre à sec. On chromatographie le résidu sur 1,5 l de silice en éluant avec un mélange de dichlorométhane et de méthanol (98-2). On obtient 8,6 g de 2-[3-(quinol-2-yl méthoxy)phényl]éthanol.Rendement : 53 %.

On dissout 8,6 g de 2-[3-(quinol-2-yl méthoxy)phényl]éthanol dans 200 ml de dichlorométhane. On ajoute 9,8 g de triphényl phosphine puis, à 10 °C, 1,9 g de brome dissous dans 50 ml de dichlorométhane. On agite 16 heures à température ambiante. On concentre à sec et reprend le résidu dans l'éther. On lave avec une solution de carbonate de sodium à 10 % et sèche sur sulfate de sodium. On concentre à sec. On chromatographie le résidu sur 450 g de silice en éluant au dichlorométhane. On obtient 7,7 g de 1-bromo-2-[3-(quinol-2-yl méthoxy)phényl]éthane. Rendement : 72 %.

Dans un ballon, on introduit 2,4 g de 1-bromo-2-[3-(quinol-2-yl méthoxy)phényl]éthane, 2,2 g de 3,8-diaza-1-oxa-2-oxo spiro[4,5]décane, 0,4 g d'iodure de potassium et 100 ml d'acétonitrile. On porte au reflux pendant 16 heures. On concentre à sec. On reprend le résidu dans le dichlorométhane. On lave avec une solution de carbonate de sodium à 10 % et sèche sur sulfate de sodium. On concentre à sec et cristallise le résidu dans un mélange d'éthanol et d'éther. On obtient 1,6 g de 8-{2-[3-(quinol-2-yl méthoxy)phényl]éthyl} 3,8-diaza 1-oxa 2-oxo spiro[4,5]décane $PF_{(cap)}$ : 132-136 °C. Rendement : 54 %.

**Exemple 2**

En opérant comme décrit dans l'exemple 1, a été préparé le composé objet de l'exemple suivant.

2) le 8-{2-[4-(quinol-2-yl méthoxy)phényl]éthyl} 3,8-diaza 1-oxa 2-oxo spiro[4,5]décane, $PF_{(cap)}$ : 186-189 °C.

**Exemple 5**

(R,S)-8-{2-hydroxy 2-[4-(quinol-2-yl méthoxy)phényl]éthyl}-3,8-diaza-1-oxa-2-oxo spiro [4,5]décane.

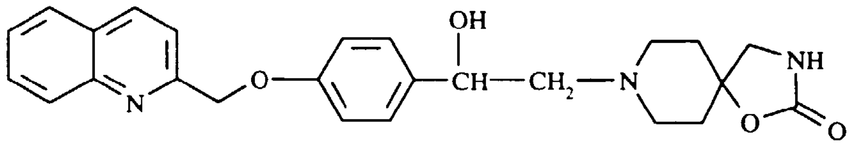

Stade A

Dans un ballon on introduit 43,5 g (0,2 mole) de 4-bromoacétyl phénol, 62,4 g (0,4 mole) de 3,8-diaza-1-oxa-2-oxo spiro[4,5]décane et 1 500 ml de méthyl éthylcétone.
On porte au reflux pendant 14 heures. On refroidit, on essore les cristaux, et les lave avec une solution à 10 % de carbonate de sodium puis à l'eau. On sèche à 50 °C sous 67 Pa. On obtient 34,8 g de 8-{2-(4-hydroxyphényl) 2-oxo éthyl}-3,8-diaza-1-oxa-2-oxo spiro[4,5]décane, $PF_{(cap)}$ : 250 °C.
Dans un appareil à hydrogéner de Parr, on introduit 20 g de 8-{2-(4-hydroxyphényl)-2-oxo éthyl]-3,8-diaza-1-oxa-2-oxo spiro[4,5]décane, 69 ml d'acide chlorhydrique N, 100 ml de méthanol, 300 ml d'eau et 7 g de Palladium sur charbon à 5 %. On hydrogène sous $7.10^5$ Pa à température ambiante. On filtre le catalyseur et concentre à sec. On reprend dans l'eau, alcalinise à la soude, essore le précipité, le lave à l'eau et le sèche à 50 °C sous 67 Pa. On obtient 14,5 g de (R, S)-8-{2-hydroxy-2-(4-hydroxyphényl)éthyl}-3,8-diaza-1-oxa-2-oxo spiro[4,5]decane, $PF_{(cap)}$ : 213-214 °C. Rendement: 72 %.

Stade B

Préparation du composé titre.

Dans un ballon tricol, on introduit 1,47 g de (R,S)-8-{2-hydroxy-2-(4-hydroxyphényl)éthyl} 3,8-diaza-1-oxa-2-oxo spiro[4,5]décane, 1,1 g de chlorhydrate de 2-chlorométhyl quinoléine, 1,53 g de carbonate de potassium et 50 ml de diméthylformamide. On agite pendant 16 heures à température ambiante puis concentre à sec. Le résidu est repris dans une solution à 10 % de carbonate de sodium. Puis on extrait au dichlorométhane, sèche sur sulfate de sodium et concentre à sec. Le résidu est chromatographié sur 200 g de silice en éluant avec un mélange dichlorométhane méthanol (95-5). On recristallise le résidu dans l'éthanol et obtient ainsi 1 g du composé titre, $PF_{(cap)}$ : 150-155 °C. Rendement : 46 %.

**Exemple 6**

(R,S) 8-{2-phényl-2-[4-(quinol-2-yl méthoxy)phényl]éthyl}-3,8-diaza-1-oxa-2-oxo spiro[4,5] décane

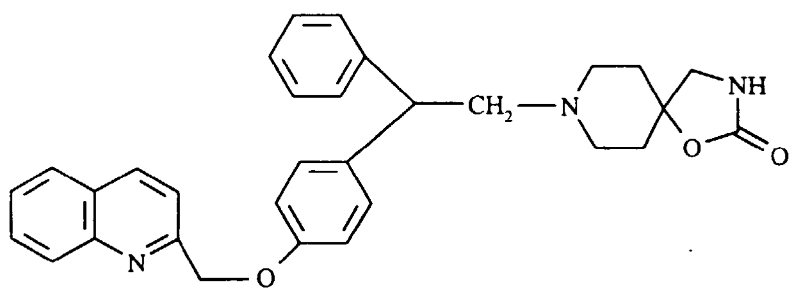

Dans un ballon, on introduit 60 g (0,3 mole) de 4-hydroxybenzophénone, 42 g de chlorure de benzyle, 45,6 g de carbonate de potassium, 0,5 g d'iodure de potassium et 1 l de diméthylformamide. On agite 72 heures à température ambiante. On filtre et concentre à sec, sans dépasser 30 °C. Le résidu est repris dans le dichlorométhane, lavé avec une solution à 10 % de carbonate de sodium puis avec une solution saturée de chlorure de lithium. On sèche sur sulfate de sodium et concentre à sec. On obtient 85 g de 4-benzyloxybenzophénone. Rendement : 99 %.
Dans un tricol, on introduit 4,8 g d'hydrure de sodium à 60 % préalablement lavé au pentane. On ajoute 26,4 g d'iodure de triméthylsulfoxonium et on coule 120 ml de diméthylsulfoxyde en 10 mn à température ambiante. On agite 30 mn et on ajoute en 10 mn 28,8 g de 4-benzyloxybenzophénone dissous dans 100 ml de diméthylsulfoxyde. On porte à 50 °C pendant 3 heures. On ajoute 300 ml d'eau et extrait à l'éther. On sèche la phase éthérée sur sulfate de sodium et concentre à sec. On reprend le résidu dans 300 ml de dichlorométhane et on coule 6,8 ml d'éthérate du

trifluorure de bore. On agite une nuit à température ambiante et hydrolyse. On lave la phase organique à l'eau et sèche sur sulfate de sodium. On concentre à sec et on chromatographie le résidu sur 530 g de silice en éluant avec un mélange dichlorométhane-cyclohexane (50-50). On obtient 10,1 g de (R,S)-2-phényl-2-(4-benzyloxyphényl) acétaldéhyde. Rendement : 33 %.

Dans un tricol, on introduit 4,5 g (0,015 mole) de (R,S) 2-phényl-2-(4-benzyloxyphényl)acetaldéhyde, 2,3 g de 3,8-diaza-1-oxa-2-oxo spiro[4,5]décane et 100 ml de dichlorométhane. On ajoute, à température ambiante, 7,05 g de triacétoxyborohydrure de sodium. On agite 16 heures, lave avec une solution à 10 % de carbonate de sodium et ajoute 100 ml d'acide chlorhydrique N. On essore et lave à l'eau puis à l'éther le précipité. On obtient 6,0 g de chlorhydrate de (R,S) 8-[2-(4-benzyloxyphényl)-2-phényl éthyl]-3,8-diaza-1-oxa-2-oxo spiro[4,5]décane.

Dans un appareil à hydrogéner de Parr, on introduit 4,0 g de chlorhydrate de (R,S) 8-[2-(4-benzyloxyphényl)-2-phényléthyl]-3,8-diaza-1-oxa-2-oxo spiro[4,5]décane, 250 ml de méthanol et 1 g d'hydroxyde de palladium sur charbon. On hydrogène à 50 °C sous $4.10^5$ Pa. On filtre le catalyseur et concentre à sec. On sèche à 20 °C/67 Pa. On obtient 4,0 g de chlorhydrate de (R,S) 8-[2-(4-hydroxyphényl)-2-phényléthyl]-3,8-diaza-1-oxa-2-oxo spiro [4,5]décane. Rendement : 92 %.

Dans un tricol, on introduit 4,0 g de chlorhydrate de (R,S) 8-[2-(4-hydroxyphényl)-2-phényléthyl]-3,8-diaza-1-oxa-2-oxo spiro[4,5]décane, 2,7 g de chlorhydrate de 2-chlorométhyl quinoléïne, 3,45 g de carbonate de potassium et 120 ml de diméthylformamide. On agite 16 heures à températures ambiante. On concentre à sec à température inférieure à 35 °C. On reprend le résidu dans le dichlorométhane. On lave avec une solution de bicarbonate de sodium puis avec une solution de chlorure de lithium. On sèche sur sulfate de sodium. On chromatographie le résidu sur 400 g de silice en éluant avec un mélange de dichlorométhane/ méthanol (97/3).
Les fractions intéressantes sont concentrées à sec. Le résidu est repris dans l'éthanol. On acidifie à pH 5 par éthanol/ acide chlorhydrique. On essore, lave à l'éthanol et sèche à 40 °C sous 67 Pa. On obtient 3,6 g de monochlorhydrate de (R,S) 8-{2-phényl-2-[4-(quinol-2-yl méthoxy)phényl]éthyl}-3,8-diaza-1-oxa-2-oxo spiro[4,5]décane. Rendement : 59 %. $PF_{(cap)}$ : 242-244 °C.

**Exemples 7 à 21**

En opérant comme décrit dans l'exemple 6, ont été préparés les composés objet des exemples suivants.

7) le (R,S) 8-{2-[4-(napht-2-yl méthoxy)phényl]-2-phényléthyl}-3,8-diaza-1-oxa-2-oxo spiro[4,5]décane, $PF_{(cap)}$ : 150-152 °C.

8) le dichlorhydrate de (R,S) 8-{2-(4-chlorophényl)-2-[4-(quinol-2-yl méthoxy)phényl]éthyl}-3,8-diaza-1-oxa-2-oxo spiro[4,5]décane, (lyophilisat).

9) le (R,S) 8-{2-phényl-2-[4-(5-phénylpyrid-2-yl méthoxy)phényl]éthyl}-3,8-diaza 1-oxa-2-oxo spiro[4,5]décane, $PF_{(cap)}$ : 166-168 °C.

10) le dichlorhydrate de (R,S) 8-{2-phényl-2-[4-(quinol-2-yl méthoxy)phényl]éthyl}-8-aza-1,4-dioxa spiro[4,5]décane, $PF_{(cap)}$ : 110 °C (déc).

11) le (R,S) 8-{2-phényl-2-[4-(quinol-2-yl méthoxy)phényl]éthyl}-1,8-diaza-3-oxa-2-oxo spiro[4,5]décane, $PF_{(cap)}$ : 160-162 °C.

12) le (R,S)-3-(2-phényléthyl) 8-{2-phényl-2-[4-(quinol-2-yl-méthoxy)phényl]éthyl}-3,8-diaza-1-oxa-2-oxo spiro [4,5]décane, $PF_{(cap)}$ : 110-112 °C.

13) le (R,S), (R,S)-4-méthyl-8-{2-phényl-2-[4-(quinol-2-yl méthoxy)phényl]éthyl}-3,8-diaza-1-oxa 2-oxo spiro[4,5] décane, $PF_{(cap)}$ : 156-158 °C.

14) le (R,S) 8-{2-[4-(6-fluoroquinol-2-yl méthoxy)phényl]-2-phényléthyl}-3,8-diaza-1-oxa 2-oxo spiro[4,5]décane, $PF_{(cap)}$ : 166-168 °C.

15) le trichlorhydrate de (R,S) 3-[2-(diméthylamino)éthyl]-8-{2-phényl-2-[4-(quinol-2-yl méthoxy)phényl]éthyl}-3,8-diaza-1-oxa-2-oxo spiro[4,5]décane, $PF_{(cap)}$ : 175 °C (déc).

16) le(R,S)-3-méthyl-8-{2-phényl-2-[4-(quinol-2-ylméthoxy)phényl]éthyl}-3,8-diaza-1-oxa-2-oxo spiro[4,5]décane, $PF_{(cap)}$ : 120-122 °C.

17) le (R,S) 3-éthyl-8-{2-phényl-2-[4-(quinol-2-yl méthoxy)phényl]éthyl}-3,8-diaza-1-oxa-2-oxo spiro[4,5]décane, $PF_{(cap)}$ : 124-126 °C.

18) le (R,S) 8-{2-phényl-2-[4-(quinol-2-yl méthoxy)phényl]éthyl}-2,8-diaza-1,3-dioxo spiro[4,5]décane, $PF_{(cap)}$ : 114-116 °C.

19) le (R,S) 8-{2-phényl-2-[4-(quinol-2-yl méthoxy)phényl]éthyl}-3,8-diaza-1-oxa-2-thioxo spiro[4,5]décane, $PF_{(cap)}$ : 198-200 °C.

21) le (R,S) 2-méthyl-8-{2-phényl-2-[4-(quinol-2-yl méthoxy)phényl]éthyl}-2,8-diaza-1,3-dioxo spiro[4,5]décane, $PF_{(cap)}$ 150-152 °C.

**Exemple 22**

Dichlorhydrate de (R,S) 8-{(4-chlorophényl) [4-(quinol-2-yl méthoxy)phényl]méthyl}-3,8-diaza 1-oxa 2-oxo spiro[4,5]décane

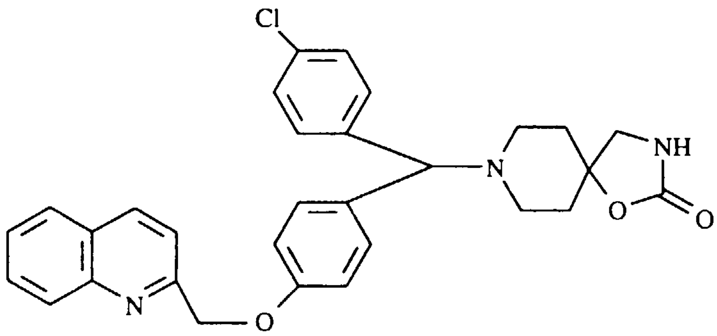

, 2 HCl

Dans un tricol, on introduit 32 g de 4-chloro-4'-benzyloxy benzophénone et 350 ml de tétrahydrofurane. On refroidit à 5 °C et on ajoute 7,6 g de borohydrure de sodium dissous dans 20 ml d'eau. On agite 16 heures à 20 °C. On concentre à sec. On reprend le résidu dans le dichlorométhane, on lave avec une solution de bicarbonate de sodium et on sèche sur sulfate de sodium. On concentre à sec et obtient 32 g de (R,S)(4-benzyloxy)(4-chlorophényl)méthanol. Rendement : 100 %. Dans un tricol, on introduit 11 g (0,034 mole) de (R,S)(4-benzyloxy)(4-chlorophényl)méthanol et 200 ml de chlorure de thionyle. On porte une heure à 50 °C. On concentre à sec. On ajoute du toluène et concentre à nouveau de façon à éliminer le chlorure de thionyle en excès. On ajoute 11,7 g de 3,8-diaza 1-oxa 2-oxo spiro[4,5] décane et 150 ml de toluène au résidu. On agite pendant 16 heures au reflux. On concentre à sec. On reprend dans du dichlorométhane et on lave avec une solution de bicarbonate de sodium. On sèche sur sulfate de sodium et concentre à sec. On obtient 12 g de (R,S) 8-[(4-chlorophényl)(4-benzyloxyphényl)méthyl]-3,8-diaza-1-oxa-2-oxo spiro[4,5] décane. Rendement : 75 %.

Dans un tricol, on introduit 12 g de (R,S) 8-[(4-chlorophényl)(4-benzyloxyphényl)méthyl]-3,8-diaza-1-oxa-2-oxo spiro[4,5]décane, 100 ml de dichlorométhane et 8,7 g de N-éthyl morpholine. On refroidit à 5 °C et on coule 55 ml de tribromure de bore dans le dichlorométhane 1N. On agite 5 heures à température ambiante et on ajoute à nouveau 55 ml de tribromure de bore dans le dichlorométhane. On agite 24 heures à température ambiante. On refroidit à 5 °C et hydrolyse par une solution de bicarbonate de sodium jusqu'à pH alcalin. On sèche la phase organique sur sulfate de sodium et concentre à sec. Le résidu est chromatographié sur 760 g de silice en éluant avec un mélange de dichlorométhane/méthanol (95/5). On obtient 3,5 g de (R,S) 8-[4-chlorophényl (4-hydroxyphényl)méthyl]-3,8-diaza-1-oxa-2-oxo spiro[4,5]décane. Rendement : 34 %.

Dans un tricol, on introduit 3,5 g de (R,S) 8-[(4-chlorophényl (4-hydroxyphényl)méthyl]-3,8-diaza-1-oxa-2-oxo spiro[4,5]décane, 3 g de carbonate de potassium, 0,2 g d'iodure de potassium, 2,3 g de chlorhydrate de 2-chlorométhyl quinoléine et 120 ml de diméthylformamide. On agite 16 heures à 60 °C. On concentre à sec. On reprend le résidu dans le dichlorométhane, lave avec un solution à 10 % de carbonate de sodium puis avec une solution saturée de chlorure de lithium. On sèche la phase organique sur sulfate de sodium. Le résidu est chromatographié sur 760 g de silice en éluant avec un mélange de dichlorométhane et de méthanol (97/3). Les fractions intéressantes sont concentrées à sec. On ajoute au résidu de l'éthanol chlorhydrique. On observe l'apparition de cristaux qui sont essorés, lavés à l'éther et séchés à 40 °C sous 67 Pa. On obtient 4,8 g de dichlorhydrate de (R,S)-8-{(4-(chlorophényl)[4-quinol-2-yl méthoxy)phényl]méthyl}-3,8-diaza-1-oxa-2-oxo spiro[4,5] décane, $PF_{(cap)}$ : 214-216 °C. Rendement : 84 %.

**Exemples 23 à 27**

En opérant comme décrit dans l'exemple 22, ont été préparés les composés objet des exemples suivants.

23) le (R,S) 8-{[4-(napht-2-yl méthoxy)phényl]phénylméthyl}-3,8-diaza-1-oxa-2-oxo spiro[4,5] décane, PF(cap) : 126-128 °C.

24) le (R,S) 8-{phényl-[4-(quinol-2-yl méthoxy)phényl]méthyl}-3,8-diaza-1-oxa-2-oxo spiro [4,5]décane, PF(cap) : 184-186°C.

26) le (R,S) 8-{phényl[4-(5-phényl pyrid-2-yl méthoxy)phényl]méthyl}-3,8-diaza-1-oxa-2-oxo spiro[4,5]décane, PF : 190-192 °C.

27) le (R,S) 8-{phényl-[4-(6-fluoro quinol-2-yl méthoxy)phényl]méthyl}-3,8-diaza-1-oxa-2-oxo spiro[4,5]décane, PF (cap) : 200-202 °C.

**Exemple 28**

8-{2-oxo-2-[4-(quinol-2-yl méthoxy)phényl]éthyl}-3,8-diaza-1-oxa-2-oxo spiro[4,5]décane

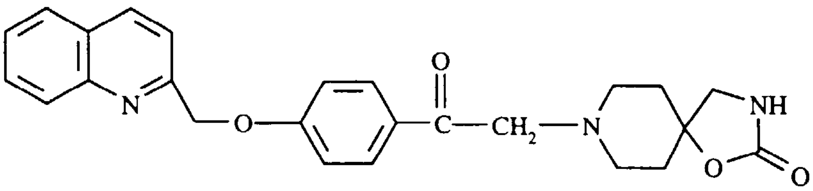

Dans un ballon, on introduit 2,9 g du 8-[2-(4-hydroxyphényl)-2-oxo]éthyl]-3,8-diaza-1-oxa-2-oxo spiro[4,5] décane, 2,14 g de chlorhydrate de 2-chlorométhylquinoléïne, 3 g de carbonate de potassium, 0,1 g d'iodure de potassium et 100 ml de diméthylformamide. On agite 24 heures à température ambiante. On concentre à sec sans chauffer. Le résidu est repris dans le dichlorométhane. On lave avec une solution à 10 % de bicarbonate de sodium, puis avec une solution saturée de chlorure de lithium. On sèche sur sulfate de sodium et concentre à sec. On chromatographie le résidu sur 350 g de silice en éluant avec un mélange de dichlorométhane/méthanol (97/3). Les fractions intéressantes sont concentrées à sec et triturées dans l'éthanol. On essore et sèche les cristaux. On obtient 1,5 g de 8-{2-oxo-2-[4-(quinol-2-yl méthoxy)phényl]éthyl}-3,8-diaza-1-oxa-2-oxo spiro[4,5]décane, PF(cap) : 166-170°C. Rendement : 35 %.

**Exemple 29**

8-{[4-(quinol-2-yl méthoxy)phényl]acétyl}-3,8-diaza-1-oxa-2-oxo spiro[4,5]décane

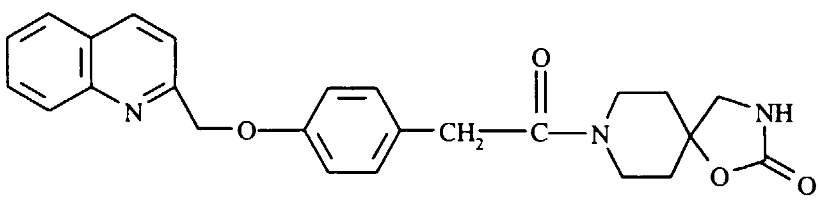

Dans un tricol, on introduit 4,9 g d'acide 4-benzyloxy phényl acétique (0,02 mole), 3,2 g de 3,8-diaza-1-oxa-2-oxo spiro[4,5]décane, 12,7 ml de N-éthylmorpholine et 100 ml de diméthylformamide. On refroidit à 5 °C. On coule 13,6 ml d'anhydride cyclique de l'acide 1-propane phosphonique 50 % dans le dichlorométhane (LANCASTER Réf: 11911). On agite 2 heures à 5 °C puis 16 heures à température ambiante. On ajoute de l'eau, extrait au dichlorométhane, lave avec une solution à 10 % de bicarbonate de sodium, puis avec une solution saturée de chlorure de lithium. On sèche sur sulfate de sodium et concentre à sec. On obtient 7,8 g de 8-{[4-benzyloxyphényl)acétyl]-3,8-diaza-1-oxa-2-oxo spiro[4,5]décane. Rendement : 100 %.

On hydrogène 7,8 g de 8-[(4-benzyloxyphényl)acétyl]-3,8-diaza-1-oxa-2-oxo spiro[4,5]décane dans 200 ml d'éthanol en présence de 1,5 g d'hydroxyde de palladium sur charbon à 50 °C sous $5.10^5$ Pa. On filtre le catalyseur et concentre à sec. On recristallise le résidu dans 150 ml d'éthanol. On obtient 3,0 g de 8-[(4-hydroxyphényl)acétyl]-3,8-diaza-1-oxa-2-oxo spiro[4,5] décane. Rendement : 50 %.

Dans un tricol, on introduit 2,7 g (0,0093 mole) de 8-{(4-hydroxyphényl)acétyl]-3,8-diaza-1-oxa-2-oxo spiro [4,5]décane, 2,2 g de chlorhydrate de 2-chlorométhyl quinoléine, 2,8 g de carbonate de potassium, 0,1 g d'iodure de potassium et 100 ml de diméthylformamide. On agite pendant 16 heures à 40 °C. On concentre à sec. On reprend le résidu dans le dichlorométhane. On lave avec une solution de bicarbonate de sodium à 10 % puis avec une solution de chlorure de lithium saturée. On sèche sur sulfate de sodium et concentre à sec. On chromatographie le résidu sur 480 g de silice en éluant avec un mélange dichlorométhane/méthanol (97/3). On concentre à sec les fractions intéressante et on triture le résidu dans l'éthanol.. On essore et sèche les cristaux. On obtient 3,0 g de 8-{[4-(quinolin-2-yl méthoxy)phényl]acétyl}-3,8-diaza-1-oxa-2-oxo spiro[4,5]décane, PF(cap) : 182-184 °C. Rendement : 75 %.

**Exemple 30**

En opérant par analogie avec l'exemple 20 a été préparé le composé suivant.

Le 8-[4-(quinol-2-yl méthoxy)benzoyl]-3,8-diaza-1-oxa-2-oxo spiro[4,5]décane, PF(cap) : 188-190 °C.

**Exemple 31**

Chlorhydrate de (R,S) 8-[2-(4-benzyloxyphényl)-3-phényl propyl]-3,8-diaza-1-oxa-2-oxo spiro[4,5]décane

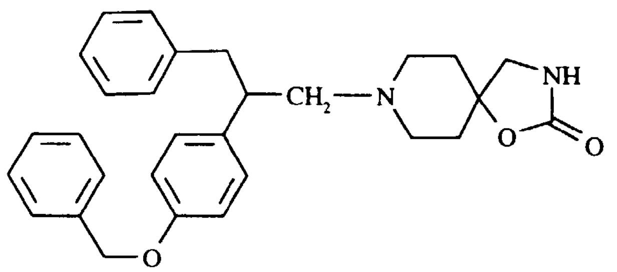

, HCl

Dans un tricol, on introduit 200 ml d'ammoniac liquide et 0,9 g de sodium. On ajoute un cristal de chlorure férrique et on agite jusqu'à disparition de la coloration bleue. On ajoute 5 g de chlorure de benzyle dissous dans 50 ml d'éther. On agite pendant 24 heures jusqu'à distillation complète de l'ammoniac. On reprend le résidu dans l'éther, lave à l'eau et sèche sur sulfate de sodium. On concentre à sec et chromatographie le résidu sur 480 g de silice en éluant avec un mélange de dichlorométhane/cyclohexane (50/50). On obtient 9,8 g de 2-(4-benzyloxyphényl)-3-phényl propionate d'éthyle. Rendement : 63 %.

Dans un tricol, on introduit 9,5 g (0,026 mole) de 2-(4-benzyloxyphényl)-3-phényl propionate d'éthyle, 40 ml de toluène et 30 ml de dichlorométhane. On refroidit à - 70 °C. On coule 44 ml d'hydrure de diisobutylaluminium 1M dans le toluène en 15 mn. On agite 1 heure à cette température. On coule à - 70 °C 30 ml de méthanol et 60 ml d'eau. On filtre l'alumine et extrait la phase aqueuse du dichlorométhane. Les phases organiques jointes sont séchées sur sulfate de sodium et concentrées à sec. On obtient 8,2 g de 2-benzyloxyphényl-3-phényl propionaldéhyde. Rendement : 100 %.

Dans un tricol, on introduit 8,0 g de 2-benzyloxyphényl-3-phényl propionaldéhyde, 4 g de 3,8-diaza-1-oxa-2-oxo spiro[4,5]décane, 12 g de triacétoxyborohydrure de sodium et 300 ml de dichlorométhane. On agite 16 heures à température ambiante. On lave avec une solution saturée de bicarbonate de sodium et sèche sur sulfate de sodium. On agite cette phase organique en présence d'acide chlorhydrique N en excès. On essore et lave à l'éthanol. On obtient 8 g de chlorhydrate de (R,S) 8-[2-(4-benzyloxyphényl)-3-phénylpropyl]-3,8-diaza-1-oxa-2-oxo spiro[4,5]décane, PF(cap) : 234-236 °C. Rendement : 66 %.

**Exemple 32**

(R,S) 8-{3-phényl 2-[4-(quinol-2-yl méthoxy)phényl]propyl}-3,8-diaza-1-oxa-2-oxo spiro [4,5]décane

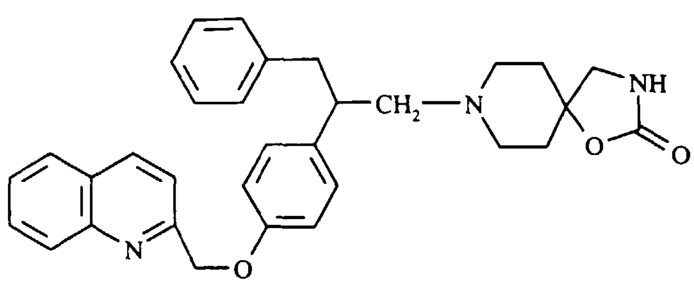

On hydrogène 7 g de chlorhydrate de (R,S) 8-[2-(4-benzyloxyphényl)-3-phényl propyl]-3,8-diaza-1-oxa-2-oxo spiro[4,5]décane (voir exemple 22) dans 400 ml de méthanol et 1,5 g d'hydroxyde de palladium sur charbon sous $5.10^5$ Pa à 45 °C. On filtre et concentre à sec. On obtient 6,1 g de chlorhydrate de (R,S) 8-[2-(4-hydroxyphényl)-3-phénylpropyl]-3,8-diaza-1-oxa-2-oxo spiro[4,5]décane. Rendement : 100 %.

Dans un tricol, on introduit 3,2 g (0,008 mole) de chlorhydrate de (R,S) 8-[2-(4-hydroxyphényl)-3-phénylpropyl]-3,8-diaza-1-oxa-2-oxo spiro[4,5]décane, 1,8 g de chlorhydrate de 2-chlorométhyl quinoléine, 3,7 g de carbonate de potassium, 0,25 g d'iodure de potassium et 100 ml de diméthyl formamide. On agite 16 heures à 60 °C. On concentre à sec. On reprend dans le dichlorométhane et on lave avec une solution à 10 % de carbonate de sodium puis avec une solution saturée de chlorure de lithium. On sèche sur sulfate de sodium et on concentre à sec. On chromatographie

le résidu sur 750 g de silice en éluant avec un mélange de dichlorométhane et de méthanol (97/3). On obtient 2,5 g de (R,S) 8-{3-phényl-2-[4-(quinol-2-yl méthoxy)phényl]propyl}-3,8-diaza-1-oxa-2-oxo spiro[4,5]décane, PF(cap) : 154-156 °C. Rendement : 69 %.

**Exemples 33 à 42**

En opérant par analogie avec l'exemple 32 ont été préparés les composés suivants.

33) le (R,S) 8-{4-phényl-2-[4-(quinol-2-yl méthoxy)phényl]butyl}-3,8-diaza-1-oxa-2-oxo spiro[4,5]décane, PF (cap) : 72-74 °C.
34) le chlorhydrate de (R,S) 8-{3-(4-chlorophényl)-2-[4-(quinol-2-yl méthoxy)phényl] propyl}- 3,8-diaza-1-oxa-2-oxo spiro[4,5]décane, PF(cap) : 176-180 °C.
35) le (R,S) 8-{3-(4-chlorophényl)-2-[4-(5-phénylpyrid-2-yl méthoxy)phényl]propyl}-3,8-diaza-1-oxa-2-oxo spiro [4,5]décane, PF(cap) : 172-174 °C.
36) le chlorhydrate de (R,S) 8-{2-cyclopentyl 2-[4-(quinol 2-yl méthoxy)phényl]éthyl}-3,8-diaza-1-oxa-2-oxo spiro [4,5]décane, PF(cap) : 240-242 °C.
37) le (R,S) 8-{3-(4-chlorophényl)-2-[4-(6-fluoroquinol-2-yl méthoxy)phényl]propyl}-3,8-diaza-1-oxa-2-oxo spiro [4,5]décane, PF(cap) : 174-176 °C.
38) le (R,S) 8-{4-(4-chlorophényl)-2-[4-(6-fluoroquinol-2-yl méthoxy)phényl]butyl}-3,8-diaza-1-oxa-2-oxo spiro [4,5]décane, PF(cap) : 173-175 °C.
39) le (R,S) 8-{4-(4-chlorophényl)-2-[4-(quinol-2-yl méthoxy)phényl]butyl}-3,8-diaza-1-oxa-2-oxo spiro[4,5]décane, PF(cap): 175-177 °C.
40) le (R,S) 8-{3-phényl-2-[4-(quinol-2-yl méthoxy)phényl]propyl}-3,8-diaza-1-oxa-2-thioxo spiro[4,5]décane, PF (cap) : 180-185 °C.
41) le (R,S) 8-{3-phényl-2-[4-(quinol-2-yl méthoxy)phényl]propyl}-2,8-diaza-3-oxo spiro [4,5]décane, PF(cap) : 154-156 °C.
42) le (R,S) 2-phényléthyl-8-{3-phényl-2-[4-(quinol-2-yl méthoxy)phényl]propyl}-2,8-diaza-1,3-dioxo spiro[4,5]décane, PF(cap) : 134-136 °C.

**Exemple 43**

Chlorhydrate de (R,S) 8-{cyclopentyl[4-(quinol-2-yl méthoxy)phényl]acétyl}-3,8-diaza-1-oxa-2-oxo spiro[4,5] décane.

Dans un tricol, on introduit 3,6 g (0,01 mole) d'acide [4-(quinol-2-yl méthoxy)phényl] cyclopentyl acétique (cf brevet EP 034 45 19 Al), 1,6 g de 3,8-diaza-1-oxa-2-oxo spiro[4,5]décane, 6,4 ml de N-éthylmorpholine et 50 ml de diméthylformamide. On refroidit à 5°C et on ajoute 6,8 ml d'anhydride cyclique de l'acide 1-propane phosphonique à 50 % dans le dichlorométhane (LANCASTER Réf : 11911). On agite 2 heures à 5 °C puis 48 heures à température ambiante. On concentre à sec et on reprend dans le dichlorométhane. On lave avec une solution saturée de bicarbonate de sodium puis avec une solution saturée de chlorure de lithium. On sèche sur sulfate de sodium et concentre à sec. On chromatographie le résidu sur 240 g de silice en éluant avec un mélange de dichlorométhane et de méthanol (97/3). On concentre à sec les fractions intéressantes et on dissout le résidu dans l'éthanol. On ajoute un léger excès d'acide chlorhydrique N. On observe une cristallisation. On essore les cristaux et sèche à 60 °C sous 67 Pa. On obtient 3,2 g de chlorhydrate de (R,S) 8-{cyclopentyl [4-(quinol-2-yl méthoxy)phényl]acétyl}-3,8-diaza-1-oxa-2-oxo spiro[4,5] décane, $PF_{(cap)}$ : 200-204 °C. Rendement : 60 %.

**Exemple 44**

ETUDE PHARMACOLOGIQUE

1/ **Méthodologie**

1.1 Détermination de l'activité inhibitrice 5-Lipoxygénase in vitro *(cf. Goldyne M.E., Burrish G.F., Poubelle P. and Borgeat P., Arachidonic Acid Metabolism among human mononuclear leukocytes. The Journal of Biological Chemistry. Vol 259, N° 4, p 8815-8819, 1984* et *New Chang C. and Gin Wensu. Stimulation of 5-Lipoxygenase Activity in Polymorphonuclear Leukocytes of Rats by Caséinate Treatment. Biochemical Pharmacology. Vol. 36, N° 8, p 3033-3036, 1987).*

La détermination des activités inhibitrices 5-Lipoxygénase a été effectuée sur leucocytes péritonéaux de rat. Une leucocytose est induite, chez les rats mâles OFA (IFFA CREDO) de 200-250 g par injection intrapéritonale de

caséinate de Sodium à 12 %. 18 heures après, les cellules sont recueillies par lavage de la cavité abdominale. Le nombre de cellules est ajusté à $2,10^6$/ml.

La suspension cellulaire est incubée en présence de $Ca^{2+}$ $5.10^{-4}$ M et $Mg^{2+}$ $2.10^{-3}$ M et de différentes concentrations des produits étudiés pendant 10 min à 37 °C. Les cellules sont ensuite stimulées avec le ionophore calcique A23187 à la concentration de $10^{-6}$ M pendant 5 min à 37 °C. Des contrôles sont réalisés en incubant les cellules avec le solvant des produits : DMSO, 2 %.

Après arrêt de la réaction à +4 °C et centrifugation, la quantité de LTB4 en ng/$2.10^6$ cellules dans les surnageants est déterminée par méthode E.I.A. (Stallergenes France). Le pourcentage d'inhibition est calculé comme suit :

$$\% \text{ inhibition} = \frac{\text{ng de LTB4 (contrôles solvant) - ng de LTB4 (pour une concentration de produit)}}{\text{ng de LTB4 (contrôles solvant)}} \times 100$$

1.2 Détermination de l'activité inhibitrice 5-Lipoxygénase ex vivo (cf. *Brideau C., Chan C., Charleson S., Denis D. et al. Pharmacology of MK-0591 (3-[1-(4-chlorobenzyl)-3-(t-butylthio)-5(quinolin-2-yl-methoxy)-indol-2-yl]-2,2-dimethyl propanoic acid), a Potent, Orally Active Leukotriene Biosynthesis Inhibitor. Can. J. Physiol. Pharmacol. Vol. 70, p. 799-807, 1992).*

L'inhibition de la biosynthèse des leucotriènes ex vivo après administration orale des composés a été déterminée par étude de la production de leucotriènes sur sang total de rats stimulé in vitro au ionophore calcique au temps 1 heure après administration orale des composés.

Les différents produits sont administrés en suspension dans l'hydroxy éthyl cellulose : H.E.C 0,5 % à la dose de 60 µmol/kg. Chaque produit est étudié sur un lot de 8 animaux différents. Un groupe contrôle reçoit H.E.C 0,5 % uniquement. Après prélèvement intracardiaque du sang de chaque rat (rats mâles Sprague Dawley de 150-200 g IFFA CREDO), 1 ml de sang hépariné est incubé avec le ionophore calcique A23187 à la concentration de $5.10^{-5}$ M pendant 10 min à 37 °C. Le sang est ensuite immédiatement centrifugé et 100 µl de plasma sont extraits dans 400 µl de méthanol. Après centrifugation, le surnageant est évaporé à sec sous $N_2$, le résidu sec est repris dans du tampon et le LTB4 est dosé par méthode E.I.A (Stallergenes France).

Le pourcentage d'inhibition est exprimé comme suit :

$$\% \text{ inhibition} = \frac{\text{ng de LTB4 / 1 ml de sang (groupe contrôle) - ng de LTB4 / 1 ml de sang (groupe traité)}}{\text{ng de LTB4 / 1 ml de sang (groupe contrôle)}} \times 100$$

**2/ Résultats**

Ils sont répertoriés dans les tableaux ci-après.

TABLEAU 1

| Inhibition de la synthèse du LTB4 et de la PGE2 par les PMN peritoneaux de rats *in vitro* | | | | | | | | | | | |
|---|---|---|---|---|---|---|---|---|---|---|---|
| | **LTB4** | | | | | | | **PGE2** | | | |
| | **n** | **Inhibition %** | | | | | **IC50** | **n** | **Inhibition %** | | **IC50** |
| Produits des ex | | $10^{-8}$ M | $4.10^{-8}$M | $10^{-7}$ M | $10^{-6}$ M | $10^{-5}$ M | | | $10^{-5}$ M | $10^{-4}$ M | |
| 1 | | | | | | | $2.10^{-5}$ M | | | | |
| 2 | | | | | 90% | | $6.10^{-7}$ M | 1 | | 23% | > $10^{-4}$ M |
| 5 | | | | 15 % | 95% | | $3.10^{-7}$ M | 1 | | | > $10^{-4}$ M |
| 6 | 3 | 21 % | 60% | 93 % | 100% | | $3,1.10^{-8}$ M | 1 | 3 % | 88 % | $10^{-5}$ M<<$10^{-4}$M |
| 7 | 1 | | | 36 % | | | >$10^{-6}$ M | | | | |
| 8 | 3 | 22 % | 85 % | 100% | 100% | | $1,7.10^{-8}$ M | 1 | 32 % | 72% | $10^{-5}$M<<$10^{-4}$M |
| 9 | 1 | | | | | | >$10^{-7}$ M | | | | |
| 10 | 1 | | | 28 % | | 92 % | >$10^{-7}$ M | | | | |
| 11 | 1 | | | 38% | | 76 % | >$10^{-7}$M | | | | |
| 12 | 1 | | | 53% | | 98% | $10^{-7}$ M | 1 | 92 % | 86% | <$10^{-5}$M |

TABLEAU 1 (suite)

| Inhibition de la synthèse du LTB4 et de la PGE2 par les PMN peritoneaux de rats *in vitro* | | | | | | | | | | | |
|---|---|---|---|---|---|---|---|---|---|---|---|
| | LTB4 | | | | | | | PGE2 | | | |
| | n | Inhibition % | | | | | IC50 | n | Inhibition % | | IC50 |
| Produits des ex | | $10^{-8}$ M | $4.10^{-8}$M | $10^{-7}$ M | $10^{-6}$ M | $10^{-5}$ M | | | $10^{-5}$ M | $10^{-4}$ M | |
| 13 | 1 | | | 21 % | | 91% | >$10^{-7}$ M | | | | |
| 22 | 3 | 18% | 76% | 97% | 100% | | $2.10^{-8}$ M | 1 | 6% | 4% | >$10^{-4}$ M |
| 23 | 1 | | | 9% | | | >$10^{-6}$M | | | | |
| 24 | 1 | | 56% | 100% | | | $3,6.10^{-8}$M | 1 | 19% | 44% | >$10^{-4}$ M |
| 26 | 1 | | | | | | >$10^{-7}$ M | | | | |
| 28 | 1 | | | | 37% | | >$10^{-6}$M | | | | |
| 29 | 1 | | | 30% | 18% | | >$10^{-6}$ M | | | | |
| 30 | 1 | | | | 14% | | >$10^{-6}$ M | | | | |
| 31 | 1 | | | | 16% | | >$10^{-6}$M | | | | |
| 32 | 3 | | 24 % | 78% | 100% | | $6,2.10^{-8}$ M | 1 | | 92 % | $10^{-5}$ M<<$10^{-4}$M |
| 33 | 2 | 6% | 54% | 86% | | | $4,9.10^{-8}$ M | 1 | 7% | 94% | $10^{-5}$M<<$10^{-4}$ M |
| 34 | 2 | 12% | 56% | 72% | 100% | | $3,9.10^{-8}$ M | 1 | 2% | 6% | >$10^{-4}$ M |
| 35 | 1 | | | | | | >$10^{-6}$ M | | | | |
| 36 | 1 | | | | | | >$10^{-7}$ M | | | | |
| 43 | 1 | | | 8% | | | >$10^{-7}$M | | | | |

**Conditions opératoires :**

Métabolisme de l'acide arachidonique par les PMN péritonéaux de rats stimulés 5 min in vitro par le ionophore calcique (A23187): $10^{-6}$ M.

Incubation des produits avec les cellules 10 mn avant la stimulation avec le ionophore.

Dosage du LTB4 par EIA.

**Produit de référence :**

Testé dans les mêmes conditions le fenspiride ou 8-(2-phényléthyl) 3,8-diaza 1-oxa 2-oxo spiro[4,5]décane, est inactif à la dose de $10^{-4}$ M.

TABLEAU 2

| Inhibition de la synthèse du LTB4 dans le sang total de rat ex vivo, 1h | |
|---|---|
| **Produits des exemples (60 µmol / kg PO)** | **Inhibition %** |
| 6 | 64% |
| 8 | 46 % |
| 22 | 17% |
| 24 | 41% |
| 32 | 55 % |
| 33 | 48% |
| 34 | 58 % |

**Conditions opératoires :**

Moyenne par rapport à tous les contrôles n=31

Produits administrés PO 1 h avant le prélèvement de sang n=8

TABLEAU 2 (suite)

| Inhibition de la synthèse du LTB4 dans le sang total de rat ex vivo, 1h | |
|---|---|
| **Produits des exemples (60 µmol / kg PO)** | **Inhibition %** |
| M Sang total de rat stimulé 10 mn in vitro par le ionophore calcique A23187 : 5.10-5<br>Dosage du LTB4 par EIA. | |

## Revendications

**1.** Les composés spiro hétérocycliques de formule I:

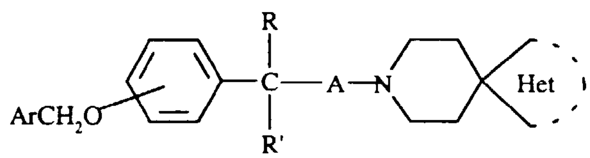

(I)

dans laquelle :

- Ar représente :

  a) un radical hydrocarboné aromatique mono- ou bi-cyclique, éventuellement substitué par un ou plusieurs substituants choisis parmi les atomes d'halogène et les radicaux $(C_1-C_5)$alkyle, $(C_1-C_5)$alkoxy, trifluorométhyle et phényle, ou
  b) un radical hétérocyclique mono- ou bi-cyclique renfermant de 1 à 3 hétéroatomes choisis parmi les atomes d'oxygène, azote et soufre et éventuellement substitué par un ou plusieurs substituants choisis parmi les atomes d'halogène et les radicaux $(C_1-C_5)$alkyle, $(C_1-C_5)$alkoxy, trifluorométhyle et phényle ;

- R représente un atome d'hydrogène ou un radical hydroxy ;
- R' représente un atome d'hydrogène ou un radical choisi parmi les radicaux $(C_1-C_5)$alkyle en chaîne droite ou ramifiée, phényle, phényl-$(C_1-C_5)$alkyle, $(C_3-C_8)$cycloalkyle et $(C_3-C_8)$cycloalkyl-$(C_1-C_5)$alkyle, chacun de ces radicaux étant soit non substitué, soit substitué par un ou plusieurs substituants choisis parmi les atomes d'halogène et les radicaux $(C_1-C_5)$alkyle, $(C_1-C_5)$alkoxy et trifluorométhyle ; ou
- R et R' représentent ensemble avec l'atome de carbone auquel ils sont liés un groupe carbonyle ;
- A représente :

  . une liaison simple,
  . un groupe carbonyle, ou
  . une chaîne hydrocarbonée droite de 1 à 5 atomes de carbone, qui peut contenir éventuellement un atome d'oxygène, et/ou être éventuellement substituée par un ou deux substituants, choisis parmi les atomes d'halogène, et les radicaux $(C_1-C_5)$alkyle, $(C_1-C_5)$alkoxy et oxo ; et

- 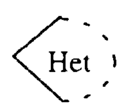

  représente un hétérocycle pentagonal contenant de 1 à 4 hétéroatomes choisis parmi les atomes d'oxygène, azote et soufre, lequel hétérocycle est soit non substitué, soit substitué par un ou deux substituants choisis parmi les radicaux $(C_1-C_5)$alkyle, $(C_1-C_5)$alkoxy, hydroxy, oxo, thio, amino, thioxo, amino$(C_1-C_5)$alkyle, $(C_1-C_5)$alkylamino$(C_1-C_5)$alkyle et di$(C_1-C_5)$alkylamino$(C_1-C_5)$alkyle,
  et, quand ils existent, les énantiomères et diastétéoisomères correspondants,
  ainsi que les sels physiologiquement tolérables des composés I avec des acides appropriés.

**2.** un composé de la revendication 1 qui est le (R, S) 8-{2-phényl-2-[4-(quinol-2-yl méthoxy)phényl]éthyl}-3,8-diaza-1-oxa-2-oxo spiro[4,5]décane, et son chlorhydrate.

**3.** Un composé de la revendication 1 qui est le (R,S) 8-{2-(4-chlorophényl)-2-[4-(quinol-2-yl méthoxy)phényl]éthyl} 3,8-diaza 1-oxa 2-oxo spiro[4,5]décane, et son dichlorhydrate.

**4.** Un composé de la revendication 1 qui est le (R,S) 8-{(4-chlorophényl) [4-(quinol-2-yl méthoxy)phényl]méthyl}-3,8-diaza-1-oxa-2-oxo spiro[4,5]décane, et son dichlorhydrate.

**5.** Un composé de la revendication 1 qui est le (R,S) 8-{phényl[4-(quinol-2-yl méthoxy)phényl]méthyl}-3,8-diaza-1-oxa-2-oxo spiro[4,5]décane.

**6.** Un composé de la revendication 1 qui est le (R,S) 8-{3-phényl 2-[4-(quinol-2-yl méthoxy)phényl]propyl}-3,8-diaza-1-oxa-2-oxo spiro[4,5]décane.

**7.** Un composé de la revendication 1 qui est le (R,S) 8-{4-phényl 2-[4-(quinol-2-yl méthoxy) phényl]butyl}-3,8-diaza-1-oxa-2-oxo spiro[4,5]décane.

**8.** Un composé de la revendication 1 qui est le (R,S) 8-{3-(4-chlorophényl) 2-[4-(quinol-2-yl méthoxy)phényl]propyl}-3,8-diaza-1-oxa-2-oxo spiro[4,5]décane, et son chlorhydrate.

**9.** Le procédé de préparation des composés de la revendication 1, caractérisé en ce que l'on fait réagir:

A) un composé de formule II :

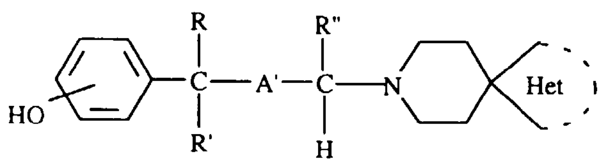

(II)

dans laquelle :

- R, R' et

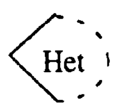

ont les significations définies dans la revendication 1,
- A' représente :
  . une liaison simple, ou
  . une chaîne hydrocarbonée droite de 1 à 4 atomes de carbone qui peut contenir éventuellement un atome d'oxygène et/ou être éventuellement substituée par un ou plusieurs substituants choisis parmi les atomes d'halogène et les radicaux ($C_1$-$C_5$)alkyle, ($C_1$-$C_5$)alkoxy, hydroxy et oxo, et
- R" représente un atome d'hydrogène ou un radical ($C_1$-$C_4$)alkyle linéaire ou ramifié ;
avec un composé de formule III :

$$Ar\text{-}CH_2\text{-}X \qquad (III)$$

dans laquelle :
- Ar a la signification définie dans la revendication 1, et

- X représente un atome d'halogène,
  pour obtenir un composé de formule Ia :

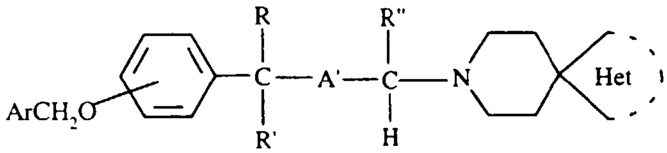

(Ia)

dans laquelle Ar, R, R', A', R" et

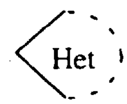

ont les significations précédemment définies ;

ou
B) un composé de formule IV :

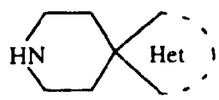

(IV)

dans laquelle

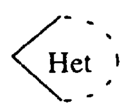

a la signification précédemment définie ;

a) soit avec un composé de formule V :

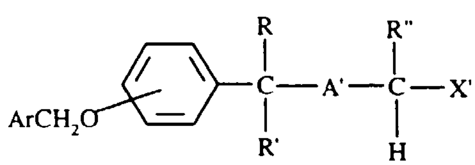

(V)

dans laquelle :

- Ar, R, R', A' et R" ont les significations précédemment définies, et
- X' représente un atome d'halogène, ou un groupe tosyloxy ou mésyloxy,
  pour obtenir un composé de formule Ia précédemment définie ;

b) soit avec un composé de formule VI :

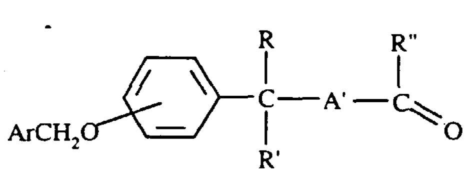

(VI)

dans laquelle Ar, R, R', A' et R" ont les significations précédemment définies, en présence d'un agent réducteur, pour obtenir un composé de formule Ia précédemment définie,
c) soit avec un composé de formule VII :

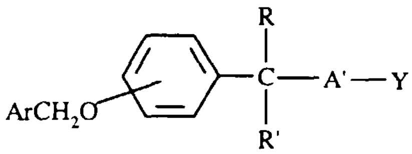

(VII)

dans laquelle :

- Ar, R, R' et A' ont les significations précédemment définies, et
- Y représente -COOH ou COCl,

pour obtenir un composé de formule Ib :

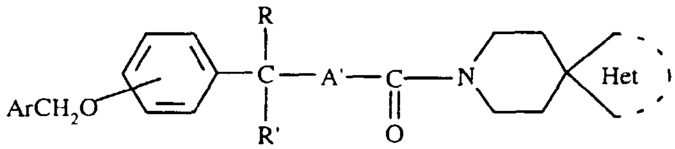

(Ib)

dans laquelle Ar, R, R', A' et

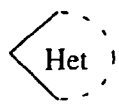

ont les significations précédemment définies ;
d) soit avec un composé de formule VIII :

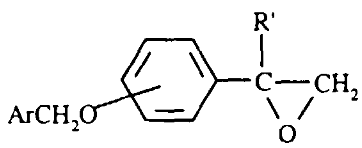

(VIII)

dans laquelle Ar et R' ont les significations précédemment définies,
pour obtenir un composé de formule Ic :

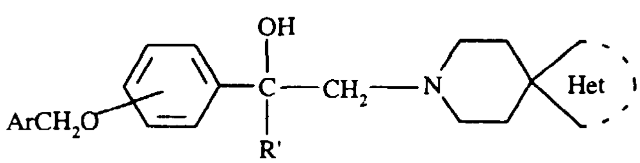

(Ic)

dans laquelle Ar, R' et

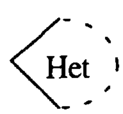

ont les significations précédemment définies.

**10.** Les compositions pharmaceutiques ayant une activité inhibitrice sur l'enzyme 5-lipoxygénase, contenant comme principe actif un composé selon l'une quelconque des revendications 1 à 8 avec un ou plusieurs excipients pharmaceutiques appropriés.

**11.** Les compositions pharmaceutiques selon la revendication 10 présentées sous une forme convenant pour le traitement de pathologies impliquant des modifications du métabolisme des leucotriènes.

**Patentansprüche**

**1.** Spiro-heterocyclische Verbindungen der Formel I:

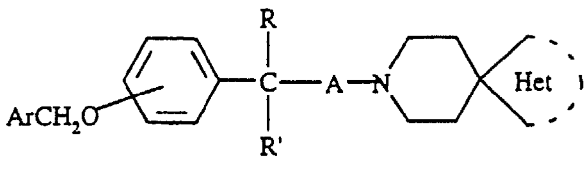

(I)

in der:

- Ar:

  a) eine mono- oder bicyclische aromatische Kohlenwasserstoffgruppe, die gegebenenfalls durch einen oder mehrere Substituenten ausgewählt aus Halogenatomen, ($C_1$-$C_5$)-Alkylgruppen, ($C_1$-$C_5$)-Alkoxygruppen, Trifluormethylgruppen und Phenylgruppen substituiert ist, oder
  b) eine mono- oder bicyclische heterocyclische Gruppe, die 1 bis 3 Heteroatome ausgewählt aus Sauerstoff-, Stickstoff- und Schwefelatomen aufweist und gegebenenfalls durch einen oder mehrere Substituenten ausgewählt aus Halogenatomen, ($C_1$-$C_5$)-Alkylgruppen, ($C_1$-$C_5$)-Alkoxygruppen, Trifluormethylgruppen und Phenylgruppen substituiert ist;

- R ein Wasserstoffatom oder eine Hydroxygruppe;
- R' ein Wasserstoffatom oder eine Gruppe ausgewählt aus geradkettigen oder verzweigten ($C_1$-$C_5$)-Alkylgruppen, Phenylgruppen, Phenyl-($C_1$-$C_5$)-alkylgruppen, ($C_3$-$C_8$)-Cycloalkylgruppen und ($C_3$-$C_8$)-Cycloalkyl-($C_1$-$C_5$)-alkylgruppen, wobei jede dieser Gruppen entweder unsubstituiert oder durch einen oder mehrere Substituenten ausgewählt aus Halogenatomen, ($C_1$-$C_5$)-Alkylgruppen, ($C_1$-$C_5$)-Alkoxygruppen und Trifluormethylgruppen substituiert ist; oder
- R und R' gemeinsam mit dem Kohlenstoffatom, an das sie gebunden sind, eine Carbonylgruppe;
- A:

  . eine Einfachbindung,
  . eine Carbonylgruppe oder
  . eine geradkettige Kohlenwasserstoffkette mit 1 bis 5 Kohlenstoffatomen, die gegebenenfalls ein Sauerstoffatom enthalten kann und/oder gegebenenfalls durch einen oder zwei Substituenten ausgewählt aus Halogenatomen, ($C_1$-$C_5$)-Alkylgruppen, ($C_1$-$C_5$)-Alkoxygruppen und Oxogruppen substituiert ist; und

- 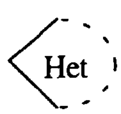

  einen fünfgliedrigen Heterocyclus, der 1 bis 4 Heteroatome ausgewählt aus Sauerstoff-, Stickstoff- und Schwefelatomen aufweist, welcher Heterocyclus entweder unsubstituiert oder durch einen oder zwei Substituenten ausgewählt aus ($C_1$-$C_5$)-Alkylgruppen, ($C_1$-$C_5$)-Alkoxygruppen, Hydroxygruppen, Oxogruppen, Thiogruppen, Aminogruppen, Thioxogruppen, Amino-($C_1$-$C_5$)-alkylgruppen, ($C_1$-$C_5$)-Alkylamino-($C_1$-$C_5$)-alkylgruppen und Di-($C_1$-$C_5$)-alkylamino-($C_1$-$C_5$)-alkylgruppen substituiert ist, bedeuten

und, falls sie existieren, die entsprechenden Enantiomeren und Diastereoisomeren, sowie die physiologisch verträglichen Salze der Verbindungen I mit geeigneten Säuren.

**2.** Verbindung nach Anspruch 1, nämlich (R,S)-8-{2-Phenyl-2-[4-(chinol-2-yl-methoxy)-phenyl]-ethyl}-3,8-diaza-1-oxa-2-oxo-spiro[4,5]decan und dessen Hydrochlorid.

**3.** Verbindung nach Anspruch 1, nämlich (R,S)-8-{2-(4-Chlorphenyl)-2-[4-(chinol-2-yl-methoxy)-phenyl]-ethyl}-3,8-diaza-1-oxa-2-oxo-spiro[4,5]decan und dessen Dihydrochlorid.

**4.** Verbindung nach Anspruch 1, nämlich (R,S)-8-{(4-Chlorphenyl)-[4-(chinol-2-yl-methoxy)-phenyl]-methyl}-3,8-diaza-1-oxa-2-oxo-spiro[4,5]decan und dessen Dihydrochlorid.

**5.** Verbindung nach Anspruch 1, nämlich (R,S)-8-{Phenyl-[4-(chinol-2-yl-methoxy)-phenyl]-methyl}-3,8-diaza-1-oxa-2-oxo-spiro[4,5]decan.

**6.** Verbindung nach Anspruch 1, nämlich (R,S)-8-{3-Phenyl-2-[4-(chinol-2-yl-methoxy)-phenyl]-propyl}-3,8-diaza-1-oxa-2-oxo-spiro[4,5]decan.

**7.** Verbindung nach Anspruch 1, nämlich (R,S)-8-{4-Phenyl-2-[4-(chinol-2-yl-methoxy)-phenyl]-butyl}-3,8-diaza-1-oxa-2-oxo-spiro[4,5]decan.

**8.** Verbindung nach Anspruch 1, nämlich (R,S)-8-(3-(4-Chlorphenyl)-2-[4-(chinol-2-yl-methoxy)-phenyl]-propyl}-3,8-diaza-1-oxa-2-oxo-spiro[4,5]decan und dessen Hydrochlorid.

**9.** Verfahren zur Herstellung der Verbindungen nach Anspruch 1, dadurch gekennzeichnet, daß man:

A) eine Verbindung der Formel (II):

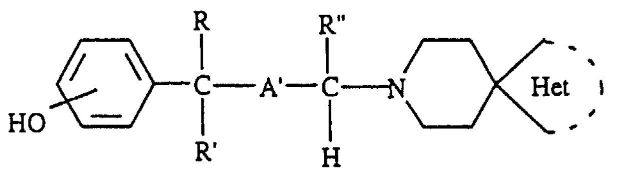

(II)

in der:

- R, R' und

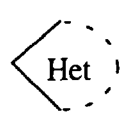

  die in Anspruch 1 angegebenen Bedeutungen besitzen,
- A':
  - . eine Einfachbindung oder
  - . eine geradkettige Kohlenwasserstoffkette mit 1 bis 4 Kohlenstoffatomen, die gegebenenfalls ein Sauerstoffatom enthalten und/oder gegebenenfalls durch einen oder mehrere Substituenten ausgewählt aus Halogenatomen, ($C_1$-$C_5$)-Alkylgruppen, ($C_1$-$C_5$)-Alkoxygruppen, Hydroxygruppen und Oxogruppen substituiert sein kann, und
- R" ein Wasserstoffatom oder eine geradkettige oder verzweigte ($C_1$-$C_4$)-Alkylgruppe bedeuten;
  mit einer Verbindung der Formel III:

$$Ar - CH_2 - X \qquad (III)$$

in der:

- Ar die in Anspruch 1 angegebenen Bedeutungen besitzt und
- X ein Halogenatom darstellt,

umsetzt zur Bildung einer Verbindung der Formel Ia:

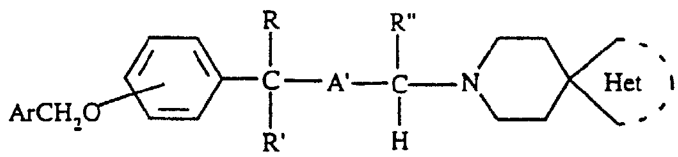

(Ia)

in der Ar, R, R', A', R" und

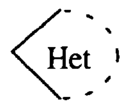

die oben angegebenen Bedeutungen besitzen;
oder

B) eine Verbindung der Formel IV:

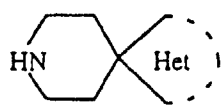

(IV)

in der

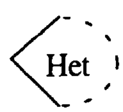

die oben angegebenen Bedeutungen besitzt;

a) entweder mit einer Verbindung der Formel V:

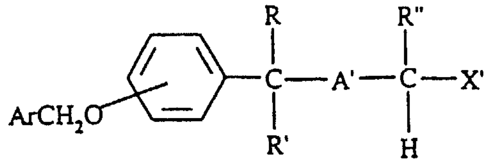

(V)

in der:

- Ar, R, R', A' und R" die oben angegebenen Bedeutungen besitzen und
- X' ein Halogenatom oder eine Tosyloxy- oder Mesyloxygruppe darstellt, umsetzt

zur Bildung einer Verbindung der oben definierten Formel Ia;

b) oder mit einer Verbindung der Formel VI:

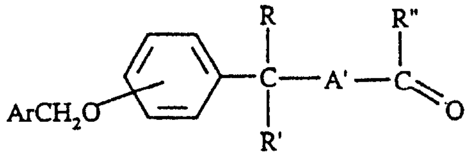

(VI)

in der Ar, R, R', A' und R" die oben angegebenen Bedeutungen besitzen, in Gegenwart eines Reduktionsmittels umsetzt zur Bildung einer Verbindung der oben definierten Formel Ia;

c) oder mit einer Verbindung der Formel VII:

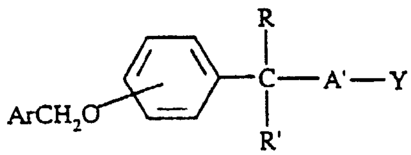

(VII)

in der:

- Ar, R, R' und A' die oben angegebenen Bedeutungen besitzen und
- Y eine Gruppe -COOH oder COCl darstellt,

umsetzt zur Bildung einer Verbindung der Formel Ib:

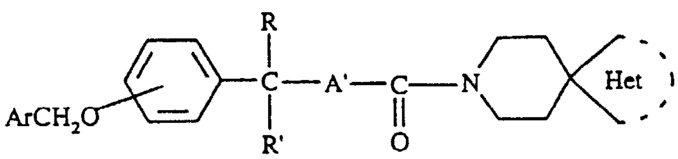

(Ib)

in der Ar, R, R', A' und

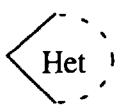

die oben angegebenen Bedeutungen besitzen;

d) oder mit einer Verbindung der Formel VIII:

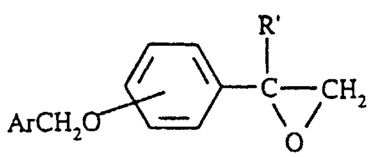

(VIII)

in der Ar und R' die oben angegebenen Bedeutungen besitzen, umsetzt zur Bildung einer Verbindung der

Formel Ic:

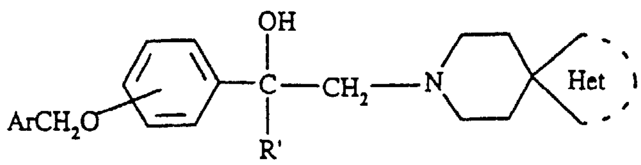

(Ic)

in der Ar, R' und

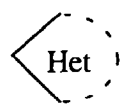

die oben angegebenen Bedeutungen besitzen.

**10.** Pharmazeutische Zubereitungen mit einer inhibierenden Wirkung auf das Enzym 5-Lipoxygenase, enthaltend als Wirkstoff eine Verbindung nach einem der Ansprüche 1 bis 8 zusammen mit einem oder mehreren pharmazeutisch geeigneten Trägermaterialien.

**11.** Pharmazeutische Zubereitungen nach Anspruch 10 in einer für die Behandlung von pathologischen Zuständen, welche Veränderungen des Leukotrien-Stoffwechsels umfassen, geeigneten Form.

**Claims**

**1.** Heterocyclic spiro compounds of formula I:

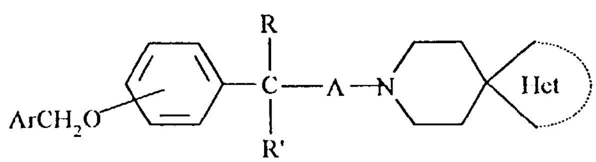

(I)

in which:

- Ar represents:

  a) a mono- or bi-cyclic aromatic hydrocarbon radical that is optionally substituted by one or more substituents selected from halogen atoms and the radicals $(C_1-C_5)$alkyl, $(C_1-C_5)$alkoxy, trifluoromethyl and phenyl, or
  b) a mono- or bi-cyclic heterocyclic radical that contains from 1 to 3 hetero atoms selected from the atoms oxygen, nitrogen and sulphur and that is optionally substituted by one or more substituents selected from halogen atoms and the radicals $(C_1-C_5)$alkyl, $(C_1-C_5)$-alkoxy, trifluoromethyl and phenyl;

- R represents a hydrogen atom or a hydroxy radical;
- R' represents a hydrogen atom or a radical selected from the radicals straight- or branched-chained $(C_1-C_5)$ alkyl, phenyl, phenyl-$(C_1-C_5)$alkyl, $(C_3-C_8)$cycloalkyl and $(C_3-C_8)$-cycloalkyl-$(C_1-C_5)$alkyl, each of those radicals being unsubstituted or substituted by one or more substituents selected from halogen atoms and the radicals $(C_1-C_5)$alkyl, $(C_1-C_5)$alkoxy and trifluoromethyl; or
- R and R' together with the carbon atom to which they are bonded represent a carbonyl group;
- A represents:

  . a single bond,

. a carbonyl group, or
. a straight hydrocarbon chain having from 1 to 5 carbon atoms that may optionally contain an oxygen atom and/or be optionally substituted by one or two substituents selected from halogen atoms and the radicals ($C_1$-$C_5$)alkyl, ($C_1$-$C_5$)alkoxy and oxo; and

-

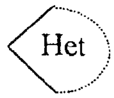

represents a pentagonal heterocycle containing from 1 to 4 hetero atoms selected from the atoms oxygen, nitrogen and sulphur, which heterocycle is unsubstituted or substituted by one or two substituents selected from the radicals ($C_1$-$C_5$)alkyl, ($C_1$-$C_5$)-alkoxy, hydroxy, oxo, thio, amino, thioxo, amino-($C_1$-$C_5$)alkyl, ($C_1$-$C_5$) alkylamino-($C_1$-$C_5$)-alkyl and di($C_1$-$C_5$)alkylamino-($C_1$-$C_5$)alkyl,
and, where they exist, the corresponding enantiomers and diastereoisomers,
as well as the physiologically tolerable salts of compounds I with suitable acids.

**2.** A compound of claim 1 which is (*R,S*)-8-{2-phenyl-2-[4-(quinol-2-ylmethoxy)phenyl]-ethyl}-3,8-diaza-1-oxa-2-oxospiro[4,5]decane, and its hydrochloride.

**3.** A compound of claim 1 which is (*R,S*)-8-{2-(4-chlorophenyl)-2-[4-(quinol-2-ylmethoxy)-phenyl]ethyl}-3,8-diaza-1-oxa-2-oxospiro[4,5]decane, and its dihydrochloride.

**4.** A compound of claim 1 which is (*R,S*)-8-{(4-chlorophenyl)-[4-(quinol-2-ylmethoxy)-phenyl]methyl}-3,8-diaza-1-oxa-2-oxospiro[4,5]decane, and its dihydrochloride.

**5.** A compound of claim 1 which is (*R,S*)-8-{phenyl-[4-(quinol-2-ylmethoxy)phenyl]-methyl}-3,8-diaza-1-oxa-2-oxospiro[4,5]decane.

**6.** A compound of claim 1 which is (*R,S*)-8-{3-phenyl-2-[4-(quinol-2-ylmethoxy)phenyl]-propyl}-3,8-diaza-1-oxa-2-oxospiro[4,5]decane.

**7.** A compound of claim 1 which is (*R,S*)-8-(4-phenyl-2-[4-(quinol-2-ylmethoxy)phenyl]-butyl}-3,8-diaza-1-oxa-2-oxospiro[4,5]decane.

**8.** A compound of claim 1 which is (*R,S*)-8-{3-(4-chlorophenyl)-2-[4-(quinol-2-ylmethoxy)-phenyl]propyl}-3,8-diaza-1-oxa-2-oxospiro[4,5]decane, and its hydrochloride.

**9.** Process for the preparation of compounds of claim 1, characterised in that:

A) a compound of formula II:

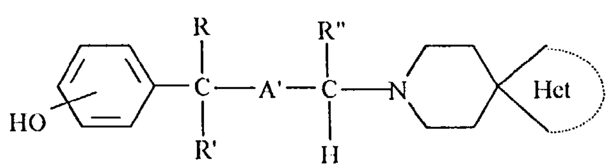

(II)

in which:

- R, R' and

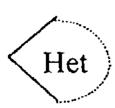

are as defined in claim 1,

- A' represents:
  - . a single bond, or
  - . a straight hydrocarbon chain having from 1 to 4 carbon atoms that may optionally contain an oxygen atom and/or be optionally substituted by one or more substituents selected from halogen atoms and the radicals $(C_1-C_5)$alkyl, $(C_1-C_5)$alkoxy, hydroxy and oxo, and
- R" represents a hydrogen atom or a linear or branched $(C_1-C_4)$alkyl radical,

is reacted with a compound of formula III:

$$Ar\text{-}CH_2\text{-}X \qquad (III)$$

in which:

- Ar is as defined in claim 1, and
- X represents a halogen atom,

to obtain a compound of formula Ia:

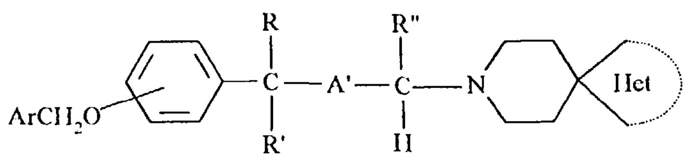

(Ia)

in which Ar, R, R', A', R" and

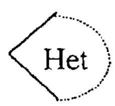

are as defined above; or

B) a compound of formula IV:

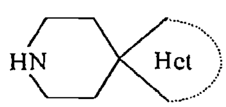

(IV)

in which

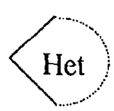

is as defined above, is reacted

a) either with a compound of formula V:

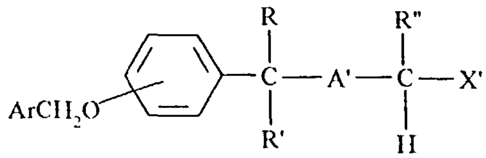

(V)

in which:

- Ar, R, R', A' and R" are as defined above, and
- X' represents a halogen atom or a tosyloxy or mesyloxy group,

to obtain a compound of formula Ia defined above;

b) or with a compound of formula VI:

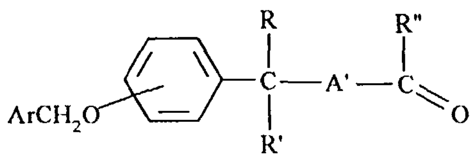

(VI)

in which Ar, R, R', A' and R" are as defined above,
in the presence of a reducing agent,
to obtain a compound of formula Ia defined above;

c) or with a compound of formula VII:

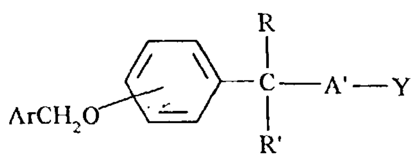

(VII)

in which:

- Ar, R, R' and A' are as defined above, and
- Y represents -COOH or COCl,

to obtain a compound of formula Ib:

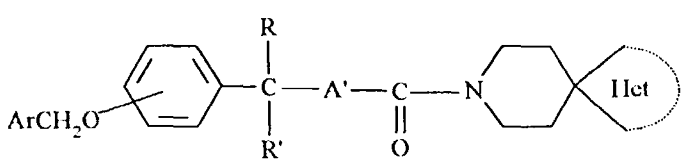

(Ib)

in which Ar, R, R', A' and

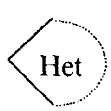

are as defined above;

d) or with a compound of formula VIII:

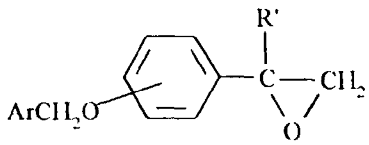

(VIII)

in which Ar and R' are as defined above, to obtain a compound of formula Ic:

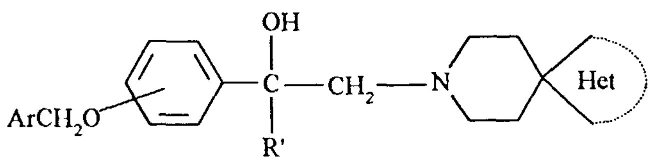

(Ic)

in which Ar, R' and

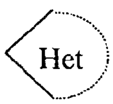

are as defined above.

**10.** Pharmaceutical compositions having inhibitory activity on the enzyme 5-lipoxygenase, comprising as active ingredient a compound according to any one of claims 1 to 8 together with one or more suitable pharmaceutical excipients.

**11.** Pharmaceutical compositions according to claim 10 presented in a form suitable for the treatment of pathologies involving modifications of leukotriene metabolism.